# EUROPEAN PATENT APPLICATION

(11) **EP 1 215 283 A1**
(43) Date of publication of application: **19.06.2002**
(21) Application number: 00939099.8
(22) Date of filing: 19.06.2000
(51) Int. Cl.: C12N 15/12, C07K 14/475, C07K 16/18

(54) **NOVEL bHLH TYPE TRANSCRIPTION FACTOR GENE DEC2**

(30) Priority: 19.08.1999 JP 23328699
(71) Applicant: CHUGAI SEIYAKU KABUSHIKI KAISHA, Tokyo, 115-8543 (JP)
(72) Inventor: FUJIMOTO, Katsumi, Hiroshima-shi, Hiroshima 734-0036 (JP); SHIN, Mei, Hiroshima-shi, Hiroshima 734-0001 (JP); KATO, Yukio, Hiroshima-shi, Hiroshima 732-0062 (JP)
(74) Representative: VOSSIUS & PARTNER
(86) International application number: JP0003991
(87) International publication number: WO0114551

(57) **Abstract**

Human- and mouse-derived bHLH type transcription factors were isolated. These transcription factors shared homology with the transcription factor DEC1, the expression of which is induced in human cartilage cells when differentiated with cAMP. Thus, these isolated transcription factors are thought to be novel transcription factors belonging to the DEC1 subfamily. The transcription factors of the present invention are also homologous to mouse Stra13 and rat SHARP that are related to neurocyte differentiation. The transcription factors of the present invention may be used as tools for developing pharmaceutical agents for diseases related to the differentiation and proliferation of cells.

SEQUENCE LISTING

<110> CHUGAI SEIYAKU KABUSHIKI KAISHA

<120> NOVEL bHLH TYPE TRANSCRIPTION FACTOR GENES DEC2

<130> C1-107PCT

<140>
<141>

<150> JP 1999-233286
<151> 1999-08-19

<160> 16

<170> PatentIn Ver. 2.1

<210> 1
<211> 3641
<212> DNA
<213> Homo sapiens

<220>
<221> CDS

<222> (135)..(1580)

<400> 1
   ctgcactgaa gagggagagc gagagagaga ctggagacgc acagatcccc ccaaggtctc 60
   ccaagcctac cgtcccacag attattgtac agagccccaa aaatcgaaac agaggaaacg 120
   aacagcagtt gaac atg gac gaa gga att cct cat ttg caa gag aga cag 170
   Met Asp Glu Gly Ile Pro His Leu Gln Glu Arg Gln
   1 5 10
   tta ctg gaa cat aga gat ttt ata gga ctg gac tat tcc tct ttg tat 218
   Leu Leu Glu His Arg Asp Phe Ile Gly Leu Asp Tyr Ser Ser Leu Tyr
   15 20 25
   atg tgt aaa ccc aaa agg agc atg aaa cga gac gac acc aag gat acc 266
   Met Cys Lys Pro Lys Arg Ser Met Lys Arg Asp Asp Thr Lys Asp Thr
   30 35 40
   tac aaa tta ccg cac aga tta ata gaa aag aaa aga aga gac cga att 314
   Tyr Lys Leu Pro His Arg Leu Ile Glu Lys Lys Arg Arg Asp Arg Ile
   45 50 55 60
   aat gaa tgc att gct cag ctg aaa gat tta ctg cct gaa cat ctg aaa 362
   Asn Glu Cys Ile Ala Gln Leu Lys Asp Leu Leu Pro Glu His Leu Lys
   65 70 75
   ttg aca act ctg gga cat ctg gag aaa gct gta gtc ttg gaa tta act 410
   Leu Thr Thr Leu Gly His Leu Glu Lys Ala Val Val Leu Glu Leu Thr
   80 85 90
   ttg aaa cac tta aaa gct tta acc gcc tta acc gag caa cag cat cag 458
   Leu Lys His Leu Lys Ala Leu Thr Ala Leu Thr Glu Gln Gln His Gln
   95 100 105
   aag ata att gct tta cag aat ggg gag cga tct ctg aaa tcg ccc att 506
   Lys Ile Ile Ala Leu Gln Asn Gly Glu Arg Ser Leu Lys Ser Pro Ile
   110 115 120
   cag tcc gac ttg gat gcg ttc cac tcg gga ttt caa aca tgc gcc aaa 554
   Gln Ser Asp Leu Asp Ala Phe His Ser Gly Phe Gln Thr Cys Ala Lys
   125 130 135 140
   gaa gtc ttg caa tac ctc tcc cgg ttt gag agc tgg aca ccc agg gag 602
   Glu Val Leu Gln Tyr Leu Ser Arg Phe Glu Ser Trp Thr Pro Arg Glu
   145 150 155
   ccg cgg tgt gtc cag ctg atc aac cac ttg cac gcc gtg gcc acc cag 650
   Pro Arg Cys Val Gln Leu Ile Asn His Leu His Ala Val Ala Thr Gln
   160 165 170
   ttc ttg ccc acc ccg cag ctg ttg act caa cag gtc cct ctg agc aaa 698
   Phe Leu Pro Thr Pro Gln Leu Leu Thr Gln Gln Val Pro Leu Ser Lys
   175 180 185
   ggc acc ggc gct ccc tcg gcc gcc ggg tcc gcg gcc gcc ccc tgc ctg 746
   Gly Thr Gly Ala Pro Ser Ala Ala Gly Ser Ala Ala Ala Pro Cys Leu
   190 195 200
   gag cgc gcg ggg cag aag ctg gag ccc ctc gcc tac tgc gtg ccc gtc 794
   Glu Arg Ala Gly Gln Lys Leu Glu Pro Leu Ala Tyr Cys Val Pro Val
   205 210 215 220
   atc cag cgg act cag ccc agc gcc gag ctc gcc gcc gag aac gac acg 842
   Ile Gln Arg Thr Gln Pro Ser Ala Glu Leu Ala Ala Glu Asn Asp Thr
   225 230 235
   gac acc gac agc ggc tac ggc ggc gaa gcc gag gcc cgg ccg gac cgc 890
   Asp Thr Asp Ser Gly Tyr Gly Gly Glu Ala Glu Ala Arg Pro Asp Arg
   240 245 250
   gag aaa ggc aaa ggc gcg ggg gcg agc cgc gtc acc atc aag cag gag 938
   Glu Lys Gly Lys Gly Ala Gly Ala Ser Arg Val Thr Ile Lys Gln Glu
   255 260 265
   cct ccc ggg gag gac tcg ccg gcg ccc aag agg atg aag ctg gat tcc 986
   Pro Pro Gly Glu Asp Ser Pro Ala Pro Lys Arg Met Lys Leu Asp Ser
   270 275 280
   cgc ggc ggc ggc agc ggc ggc ggc ccg ggg ggc ggc gcg gcg gcg gcg 1034
   Arg Gly Gly Gly Ser Gly Gly Gly Pro Gly Gly Gly Ala Ala Ala Ala
   285 290 295 300
   gca gcc gcg ctt ctg ggg ccc gac cct gcc gcc gcg gcc gcg ctg ctg 1082
   Ala Ala Ala Leu Leu Gly Pro Asp Pro Ala Ala Ala Ala Ala Leu Leu
   305 310 315
   aga ccc gac gcc gcc ctg ctc agc tcg ctg gtg gcg ttc ggc gga ggc 1130
   Arg Pro Asp Ala Ala Leu Leu Ser Ser Leu Val Ala Phe Gly Gly Gly
   320 325 330
   gga ggc gcg ccc ttc ccg cag ccc gcg gcc gcc gcg gcc ccc ttc tgc 1178
   Gly Gly Ala Pro Phe Pro Gln Pro Ala Ala Ala Ala Ala Pro Phe Cys
   335 340 345
   ctg ccc ttc tgc ttc ctc tcg cct tct gca gct gcc gcc tac gtg cag 1226
   Leu Pro Phe Cys Phe Leu Ser Pro Ser Ala Ala Ala Ala Tyr Val Gln
   350 355 360
   ccc ttc ctg gac aag agc ggc ctg gag aag tat ctg tac ccg gcg gcg 1274
   Pro Phe Leu Asp Lys Ser Gly Leu Glu Lys Tyr Leu Tyr Pro Ala Ala
   365 370 375 380
   gct gcc gcc ccg ttc ccg ctg cta tac ccc ggc atc ccc gcc ccg gcg 1322
   Ala Ala Ala Pro Phe Pro Leu Leu Tyr Pro Gly Ile Pro Ala Pro Ala
   385 390 395
   gca gcc gcg gca gcc gcc gcc gcc gct gcc gcc gcc gcc gcc gcg ttc 1370
   Ala Ala Ala Ala Ala Ala Ala Ala Ala Ala Ala Ala Ala Ala Ala Phe
   400 405 410
   ccc tgc ctg tcc tcg gtg ttg tcg ccc cct ccc gag aag gcg ggc gcc 1418
   Pro Cys Leu Ser Ser Val Leu Ser Pro Pro Pro Glu Lys Ala Gly Ala
   415 420 425
   gcc gcc gcg acc ctc ctg ccg cac gag gtg gcg ccc ctt ggg gcg ccg 1466
   Ala Ala Ala Thr Leu Leu Pro His Glu Val Ala Pro Leu Gly Ala Pro
   430 435 440
   cac ccc cag cac ccg cac ggc cgc acc cac ctg ccc ttc gcc ggg ccc 1514
   His Pro Gln His Pro His Gly Arg Thr His Leu Pro Phe Ala Gly Pro
   445 450 455 460
   cgc gag ccg ggg aac ccg gag agc tct gct cag gaa gat ccc tcg cag 1562
   Arg Glu Pro Gly Asn Pro Glu Ser Ser Ala Gln Glu Asp Pro Ser Gln
   465 470 475
   cca gga aag gaa gct ccc tgaatccttg cgtcccgaag gacggaggtt 1610
   Pro Gly Lys Glu Ala Pro
   480
   caagcagagt gagaagttaa aataccctta aggaggttca agcagagtga gaagttaaaa 1670
   tacccttaag gtctttaagg gaggaagtgt aatagatgca cgacaggcat aaacaagaac 1730
   aacaaaacag gtgttatgtg tacattcgga gttcctgttt tgctcatccc gcaccacccc 1790
   accctccaca cactaacatc cctttcttcc ccccaccagc tgtaaaagat cctatgcgaa 1850
   agacactggc tctttttttt aatcccccaa ataaattttg ccccctttta ggccatgttc 1910
   cattatctct taaaattgga acctaattcg agaggaagta agaagggtct gttctgtggc 1970
   tgagctaggt gaaccccggg gtaggggaaa gatgttaaca cctttgacgt ctttggagtt 2030
   gacatggaac agcaggtagt tgttatgtag agctagttct caaagctgcc ctgcctgttt 2090
   taggaggcgt tccacaaaca gattgaggct ctttttagaa ttgaatttac tcttcagtat 2150
   tttctaatgt tcagctttct aaaaggcata tatttttcaa agaagtgagg atgcagtttc 2210
   tcacgttgca acctattctg aagtggttta aatggtatct cttagtaact tgcactcgtt 2270
   aaagaaacac ggagctgggc catcgtcaga actaagtcag ggaaggagat ggatgagaag 2330
   gccagaatca ttcctagtac atttgctaac actttattga gaaattgacc atgaattaat 2390
   ggactcatct taatttcttc taagtccata tatagataga tatctatctg tacagatttc 2450
   tatttatcca tagataggta tctatacata cacatctcaa gtgcatctat tcccactctc 2510
   attaatccat catgttccta aatttttgta atcttactgt aaaaaaaagt gcactgaact 2570
   tcaaaacaaa acaaaaaaca acaacaacaa aaaacaagtc caaactgata tatcctatat 2630
   tctgttaaaa ttcaaaagtg aacgaaagca tttaactggc cagttttgat tgcaaatgct 2690
   gtaaagatat agaatgaagt cctgtgaggc cttcctatct ccaagtctat gtattttctg 2750
   gagaccaaac cagataccag ataatcacaa agaaagcttt tttaataagg cttaaaccaa 2810
   gaccttgtct agatattttt agtttgttgc caaggtagca ctgtgagaaa tctcacttgg 2870
   atgttatgta aggggtgaga cacaacagtc tgactatgag tgaggaaaat atctgggtct 2930
   tttcgtcagt ttggtgcatt tgctgctgct gttgctactg tttgcctcaa acgctgtgtt 2990
   taaacaacgt taaactctta gcctacaagg tggctcttat gtacatagtt gttaatacat 3050
   ccaattaatg atgtctgaca tgctattttt gtagggagaa aatatgtgct aatgatattt 3110
   tgagttaaaa tatcttttgg ggaggatttg ctgaaaagtt gcacttttgt tacaatgctt 3170
   atgcttggta caagcttatg ctgtcttaaa ttattttaaa aaaattaaat actgtctgtg 3230
   agaaaccagc tggtttagaa aagtttagta tgtgacgata aactagaaat tacctttata 3290
   ttctagtatt ttcagcactc cataaattct attacctaaa tattgccaca ctattttgtg 3350
   atttaaaaat tcttactaag gaataaaaac tttaatatac gatatgatat tgtctaataa 3410
   ttaaaaaaga cataatggat gctcaattag ttttaagata tctataacta tagggataca 3470
   aatcactaca gttctcagat ttacaccttt tttttgtcat tggcttgatg tcacacattt 3530
   ccaatctctt gcaagcctcc aggctctggc tttgtctacc tgctcgttcc caatgtatct 3590
   taatgaaaag tgcaaaagaa aaacctacca attaaaaaaa aaaaaaaaaa a 3641

<210> 2

<211> 482

<212> PRT

<213> Homo sapiens

<400> 2

Met Asp Glu Gly Ile Pro His Leu Gln Glu Arg Gln Leu Leu Glu His

1               5                   10                  15

Arg Asp Phe Ile Gly Leu Asp Tyr Ser Ser Leu Tyr Met Cys Lys Pro
            20                  25                  30
Lys Arg Ser Met Lys Arg Asp Asp Thr Lys Asp Thr Tyr Lys Leu Pro
        35                  40                  45
His Arg Leu Ile Glu Lys Lys Arg Arg Asp Arg Ile Asn Glu Cys Ile
    50                  55                  60
Ala Gln Leu Lys Asp Leu Leu Pro Glu His Leu Lys Leu Thr Thr Leu
65                  70                  75                  80
Gly His Leu Glu Lys Ala Val Val Leu Glu Leu Thr Leu Lys His Leu
                85                  90                  95
Lys Ala Leu Thr Ala Leu Thr Glu Gln Gln His Gln Lys Ile Ile Ala
            100                 105                 110
Leu Gln Asn Gly Glu Arg Ser Leu Lys Ser Pro Ile Gln Ser Asp Leu
        115                 120                 125
Asp Ala Phe His Ser Gly Phe Gln Thr Cys Ala Lys Glu Val Leu Gln
    130                 135                 140
Tyr Leu Ser Arg Phe Glu Ser Trp Thr Pro Arg Glu Pro Arg Cys Val

145                 150                 155                 160

Gln Leu Ile Asn His Leu His Ala Val Ala Thr Gln Phe Leu Pro Thr

                165                 170                 175

Pro Gln Leu Leu Thr Gln Gln Val Pro Leu Ser Lys Gly Thr Gly Ala

            180                 185                 190

Pro Ser Ala Ala Gly Ser Ala Ala Ala Pro Cys Leu Glu Arg Ala Gly

        195                 200                 205

Gln Lys Leu Glu Pro Leu Ala Tyr Cys Val Pro Val Ile Gln Arg Thr

    210                 215                 220

Gln Pro Ser Ala Glu Leu Ala Ala Glu Asn Asp Thr Asp Thr Asp Ser

225                 230                 235                 240

Gly Tyr Gly Gly Glu Ala Glu Ala Arg Pro Asp Arg Glu Lys Gly Lys

                245                 250                 255

Gly Ala Gly Ala Ser Arg Val Thr Ile Lys Gln Glu Pro Pro Gly Glu

            260                 265                 270

Asp Ser Pro Ala Pro Lys Arg Met Lys Leu Asp Ser Arg Gly Gly Gly

        275                 280                 285

Ser Gly Gly Gly Pro Gly Gly Gly Ala Ala Ala Ala Ala Ala Ala Leu
    290                 295                 300
Leu Gly Pro Asp Pro Ala Ala Ala Ala Ala Leu Leu Arg Pro Asp Ala
305                 310                 315                 320
Ala Leu Leu Ser Ser Leu Val Ala Phe Gly Gly Gly Gly Gly Ala Pro
                325                 330                 335
Phe Pro Gln Pro Ala Ala Ala Ala Ala Pro Phe Cys Leu Pro Phe Cys
            340                 345                 350
Phe Leu Ser Pro Ser Ala Ala Ala Ala Tyr Val Gln Pro Phe Leu Asp
        355                 360                 365
Lys Ser Gly Leu Glu Lys Tyr Leu Tyr Pro Ala Ala Ala Ala Ala Pro
    370                 375                 380
Phe Pro Leu Leu Tyr Pro Gly Ile Pro Ala Pro Ala Ala Ala Ala Ala
385                 390                 395                 400
Ala Ala Ala Ala Ala Ala Ala Ala Ala Ala Ala Phe Pro Cys Leu Ser
                405                 410                 415
Ser Val Leu Ser Pro Pro Pro Glu Lys Ala Gly Ala Ala Ala Ala Thr
            420                 425                 430

Leu Leu Pro His Glu Val Ala Pro Leu Gly Ala Pro His Pro Gln His
        435                 440                 445

Pro His Gly Arg Thr His Leu Pro Phe Ala Gly Pro Arg Glu Pro Gly
    450                 455                 460

Asn Pro Glu Ser Ser Ala Gln Glu Asp Pro Ser Gln Pro Gly Lys Glu
465                 470                 475                 480

Ala Pro

<210> 3
<211> 32
<212> DNA
<213> Artificial Sequence

<220>
<223> Description of Artificial Sequence:Artificially
      Synthesized Primer Sequence

<400> 3
ctattcgatg atgaagatac cccaccaaac cc                                32

<210> 4

<211> 25

<212> DNA

<213> Artificial Sequence

<220>

<223> Description of Artificial Sequence:Artificially

Synthesized Primer Sequence

<400> 4

gcaagtggtt gatcagctgg acaca 25

<210> 5

<211> 21

<212> DNA

<213> Artificial Sequence

<220>

<223> Description of Artificial Sequence:Artificially

Synthesized Primer Sequence

<400> 5

gcttacccat acgatgttcc a 21

<210> 6

<211> 26

<212> DNA

<213> Artificial Sequence

<220>

<223> Description of Artificial Sequence:Artificially

Synthesized Primer Sequence

<400> 6

tggaacgcat ccaagtcgga ctgaat 26

<210> 7

<211> 23

<212> DNA

<213> Artificial Sequence

<220>

<223> Description of Artificial Sequence:Artificially

Synthesized Primer Sequence

<400> 7

ttgaacatgg acgaaggaat tcc 23

<210> 8

<211> 32

<212> DNA

<213> Artificial Sequence

<220>

<223> Description of Artificial Sequence:Artificially

Synthesized Primer Sequence

<400> 8

gagatggtgc acgatgcaca gttgaagtga ac 32

<210> 9

<211> 26

<212> DNA

<213> Artificial Sequence

<220>

<223> Description of Artificial Sequence:Artificially

Synthesized Primer Sequence

<400> 9

attcagtccg acttggatgc gttcca 26

<210> 10

<211> 23

<212> DNA

<213> Artificial Sequence

<220>

<223> Description of Artificial Sequence:Artificially

Synthesized Primer Sequence

<400> 10

gcggggtttt tcagtatcta cga 23

<210> 11

<211> 1511

<212> DNA

<213> Homo sapiens

<220>

<221> CDS

<222> (2)..(1453)

<400> 11

c atg gac gaa gga att cct cat ttg caa gag aga cag tta ctg gaa cat 49

Met Asp Glu Gly Ile Pro His Leu Gln Glu Arg Gln Leu Leu Glu His

1                5                   10                  15

aga gat ttt ata gga ctg gac tat tcc tct ttg tat atg tgt aaa ccc    97
Arg Asp Phe Ile Gly Leu Asp Tyr Ser Ser Leu Tyr Met Cys Lys Pro
            20                  25                  30

aaa agg agc atg aaa cga gac gac acc aag gta agt gat acc tac aaa    145
Lys Arg Ser Met Lys Arg Asp Asp Thr Lys Val Ser Asp Thr Tyr Lys
        35                  40                  45

tta ccg cac aga tta ata gaa aag aaa aga aga gac cga att aat gaa    193
Leu Pro His Arg Leu Ile Glu Lys Lys Arg Arg Asp Arg Ile Asn Glu
    50                  55                  60

tgc att gct cag ctg aaa gat tta ctg cct gaa cat ctg aaa ttg aca    241
Cys Ile Ala Gln Leu Lys Asp Leu Leu Pro Glu His Leu Lys Leu Thr
65                  70                  75                  80

act ctg gga cat ctg gag aaa gct gta gtc ttg gaa tta act ttg aaa    289
Thr Leu Gly His Leu Glu Lys Ala Val Val Leu Glu Leu Thr Leu Lys
                85                  90                  95

cac tta aaa gct tta acc gcc tta acc gag caa cag cat cag aag ata    337
His Leu Lys Ala Leu Thr Ala Leu Thr Glu Gln Gln His Gln Lys Ile
            100                 105                 110

att gct tta cag aat ggg gag cga tct ctg aaa tcg ccc att cag tcc 385
Ile Ala Leu Gln Asn Gly Glu Arg Ser Leu Lys Ser Pro Ile Gln Ser
        115                 120                 125

gac ttg gat gcg ttc cac tcg gga ttt caa aca tgc gcc aaa gaa gtc 433
Asp Leu Asp Ala Phe His Ser Gly Phe Gln Thr Cys Ala Lys Glu Val
    130                 135                 140

ttg caa tac ctc tcc cgg ttt gag agc tgg aca ccc agg gag ccg cgg 481
Leu Gln Tyr Leu Ser Arg Phe Glu Ser Trp Thr Pro Arg Glu Pro Arg
145                 150                 155                 160

tgt gtc cag ctg atc aac cac ttg cac gcc gtg gcc acc cag ttc ttg 529
Cys Val Gln Leu Ile Asn His Leu His Ala Val Ala Thr Gln Phe Leu
                165                 170                 175

ccc acc ccg cag ctg ttg act caa cag gtc cct ctg agc aaa ggc acc 577
Pro Thr Pro Gln Leu Leu Thr Gln Gln Val Pro Leu Ser Lys Gly Thr
            180                 185                 190

ggc gct ccc tcg gcc gcc ggg tcc gcg gcc gcc ccc tgc ctg gag cgc 625
Gly Ala Pro Ser Ala Ala Gly Ser Ala Ala Ala Pro Cys Leu Glu Arg
        195                 200                 205

gcg ggg cag aag ctg gag ccc ctc gcc tac tgc gtg ccc gtc atc cag 673
Ala Gly Gln Lys Leu Glu Pro Leu Ala Tyr Cys Val Pro Val Ile Gln

    210                 215                 220

cgg act cag ccc agc gcc gag ctc gcc gcc gag aac gac acg gac acc 721
Arg Thr Gln Pro Ser Ala Glu Leu Ala Ala Glu Asn Asp Thr Asp Thr
225                 230                 235                 240

gac agc ggc tac ggc ggc gaa gcc gag gcc cgg ccg gac cgc gag aaa 769
Asp Ser Gly Tyr Gly Gly Glu Ala Glu Ala Arg Pro Asp Arg Glu Lys
                245                 250                 255

ggc aaa ggc gcg ggg gcg agc cgc gtc acc atc aag cag gag cct ccc 817
Gly Lys Gly Ala Gly Ala Ser Arg Val Thr Ile Lys Gln Glu Pro Pro
            260                 265                 270

ggg gag gac tcg ccg gcg ccc aag agg atg aag ctg gat tcc cgc ggc 865
Gly Glu Asp Ser Pro Ala Pro Lys Arg Met Lys Leu Asp Ser Arg Gly
        275                 280                 285

ggc ggc agc ggc ggc ggc ccg ggg ggc ggc gcg gcg gcg gcg gca gcc 913
Gly Gly Ser Gly Gly Gly Pro Gly Gly Gly Ala Ala Ala Ala Ala Ala
    290                 295                 300

gcg ctt ctg ggg ccc gac cct gcc gcc gcg gcc gcg ctg ctg aga ccc 961
Ala Leu Leu Gly Pro Asp Pro Ala Ala Ala Ala Ala Leu Leu Arg Pro
305                 310                 315                 320

gac gcc gcc ctg ctc agc tcg ctg gtg gcg ttc ggc gga ggc gga ggc 1009
Asp Ala Ala Leu Leu Ser Ser Leu Val Ala Phe Gly Gly Gly Gly Gly
                325                 330                 335

gcg ccc ttc ccg cag ccc gcg gcc gcc gcg gcc ccc ttc tgc ctg ccc 1057
Ala Pro Phe Pro Gln Pro Ala Ala Ala Ala Ala Pro Phe Cys Leu Pro
            340                 345                 350

ttc tgc ttc ctc tcg cct tct gca gct gcc gcc tac gtg cag ccc ttc 1105
Phe Cys Phe Leu Ser Pro Ser Ala Ala Ala Ala Tyr Val Gln Pro Phe
        355                 360                 365

ctg gac aag agc ggc ctg gag aag tat ctg tac ccg gcg gcg gct gcc 1153
Leu Asp Lys Ser Gly Leu Glu Lys Tyr Leu Tyr Pro Ala Ala Ala Ala
    370                 375                 380

gcc ccg ttc ccg ctg cta tac ccc ggc atc ccc gcc ccg gcg gca gcc 1201
Ala Pro Phe Pro Leu Leu Tyr Pro Gly Ile Pro Ala Pro Ala Ala Ala
385                 390                 395                 400

gcg gca gcc gcc gcc gcc gct gcc gcc gcc gcc gcc gcg ttc ccc tgc 1249
Ala Ala Ala Ala Ala Ala Ala Ala Ala Ala Ala Ala Ala Phe Pro Cys
                405                 410                 415

ctg tcc tcg gtg ttg tcg ccc cct ccc gag aag gcg ggc gcc gcc gcc 1297
Leu Ser Ser Val Leu Ser Pro Pro Pro Glu Lys Ala Gly Ala Ala Ala

            420                 425                 430

gcg acc ctc ctg ccg cac gag gtg gcg ccc ctt ggg gcg ccg cac ccc 1345
Ala Thr Leu Leu Pro His Glu Val Ala Pro Leu Gly Ala Pro His Pro
        435                 440                 445

cag cac ccg cac ggc cgc acc cac ctg ccc ttc gcc ggg ccc cgc gag 1393
Gln His Pro His Gly Arg Thr His Leu Pro Phe Ala Gly Pro Arg Glu
    450                 455                 460

ccg ggg aac ccg gag agc tct gct cag gaa gat ccc tcg cag cca gga 1441
Pro Gly Asn Pro Glu Ser Ser Ala Gln Glu Asp Pro Ser Gln Pro Gly
465                 470                 475                 480

aag gaa gct ccc tgaatccttg cgtcccgaag gacggaggtt caagcagagt 1493
Lys Glu Ala Pro

gagaagttaa aataccct 1511

<210> 12
<211> 484
<212> PRT
<213> Homo sapiens

<400> 12

Met Asp Glu Gly Ile Pro His Leu Gln Glu Arg Gln Leu Leu Glu His
1               5                   10                  15

Arg Asp Phe Ile Gly Leu Asp Tyr Ser Ser Leu Tyr Met Cys Lys Pro
            20                  25                  30

Lys Arg Ser Met Lys Arg Asp Asp Thr Lys Val Ser Asp Thr Tyr Lys
        35                  40                  45

Leu Pro His Arg Leu Ile Glu Lys Lys Arg Arg Asp Arg Ile Asn Glu
    50                  55                  60

Cys Ile Ala Gln Leu Lys Asp Leu Leu Pro Glu His Leu Lys Leu Thr
65                  70                  75                  80

Thr Leu Gly His Leu Glu Lys Ala Val Val Leu Glu Leu Thr Leu Lys
                85                  90                  95

His Leu Lys Ala Leu Thr Ala Leu Thr Glu Gln Gln His Gln Lys Ile
            100                 105                 110

Ile Ala Leu Gln Asn Gly Glu Arg Ser Leu Lys Ser Pro Ile Gln Ser
        115                 120                 125

Asp Leu Asp Ala Phe His Ser Gly Phe Gln Thr Cys Ala Lys Glu Val
    130                 135                 140

Leu Gln Tyr Leu Ser Arg Phe Glu Ser Trp Thr Pro Arg Glu Pro Arg
145                 150                 155                 160
Cys Val Gln Leu Ile Asn His Leu His Ala Val Ala Thr Gln Phe Leu
                165                 170                 175
Pro Thr Pro Gln Leu Leu Thr Gln Gln Val Pro Leu Ser Lys Gly Thr
            180                 185                 190
Gly Ala Pro Ser Ala Ala Gly Ser Ala Ala Ala Pro Cys Leu Glu Arg
        195                 200                 205
Ala Gly Gln Lys Leu Glu Pro Leu Ala Tyr Cys Val Pro Val Ile Gln
    210                 215                 220
Arg Thr Gln Pro Ser Ala Glu Leu Ala Ala Glu Asn Asp Thr Asp Thr
225                 230                 235                 240
Asp Ser Gly Tyr Gly Gly Glu Ala Glu Ala Arg Pro Asp Arg Glu Lys
                245                 250                 255
Gly Lys Gly Ala Gly Ala Ser Arg Val Thr Ile Lys Gln Glu Pro Pro
            260                 265                 270
Gly Glu Asp Ser Pro Ala Pro Lys Arg Met Lys Leu Asp Ser Arg Gly

        275                 280                 285

Gly Gly Ser Gly Gly Gly Pro Gly Gly Gly Ala Ala Ala Ala Ala Ala

    290                 295                 300

Ala Leu Leu Gly Pro Asp Pro Ala Ala Ala Ala Ala Leu Leu Arg Pro

305                 310                 315                 320

Asp Ala Ala Leu Leu Ser Ser Leu Val Ala Phe Gly Gly Gly Gly Gly

                325                 330                 335

Ala Pro Phe Pro Gln Pro Ala Ala Ala Ala Ala Pro Phe Cys Leu Pro

            340                 345                 350

Phe Cys Phe Leu Ser Pro Ser Ala Ala Ala Ala Tyr Val Gln Pro Phe

        355                 360                 365

Leu Asp Lys Ser Gly Leu Glu Lys Tyr Leu Tyr Pro Ala Ala Ala Ala

    370                 375                 380

Ala Pro Phe Pro Leu Leu Tyr Pro Gly Ile Pro Ala Pro Ala Ala Ala

385                 390                 395                 400

Ala Ala Ala Ala Ala Ala Ala Ala Ala Ala Ala Ala Ala Phe Pro Cys

                405                 410                 415

Leu Ser Ser Val Leu Ser Pro Pro Pro Glu Lys Ala Gly Ala Ala Ala

            420                 425                 430

Ala Thr Leu Leu Pro His Glu Val Ala Pro Leu Gly Ala Pro His Pro

        435                 440                 445

Gln His Pro His Gly Arg Thr His Leu Pro Phe Ala Gly Pro Arg Glu

    450                 455                 460

Pro Gly Asn Pro Glu Ser Ser Ala Gln Glu Asp Pro Ser Gln Pro Gly

465                 470                 475                 480

Lys Glu Ala Pro

<210> 13

<211> 1421

<212> DNA

<213> Mus musculus

<220>

<221> CDS

<222> (74)..(1303)

<400> 13

aaaatctctc caggcgaccg tcccacagat tacaggacag aaacctccaa atcgaaacgg 60

acagccattg aac atg gac gaa gga atc cct cat ttg caa gag aga cag 109
Met Asp Glu Gly Ile Pro His Leu Gln Glu Arg Gln
1               5                   10

tta ctg gaa cat agg gat ttt ata gga ctg gac tat tcc tct ttg tat 157
Leu Leu Glu His Arg Asp Phe Ile Gly Leu Asp Tyr Ser Ser Leu Tyr
        15                  20                  25

atg tgt aaa ccc aaa agg agc ttg aag cga gac gat acc aag gat acc 205
Met Cys Lys Pro Lys Arg Ser Leu Lys Arg Asp Asp Thr Lys Asp Thr
    30                  35                  40

tac aag tta ccg cac aga tta ata gaa aag aag aga cga gac cga att 253
Tyr Lys Leu Pro His Arg Leu Ile Glu Lys Lys Arg Arg Asp Arg Ile
45                  50                  55                  60

aat gaa tgc att gct cag ctg aaa gat tta ctg ccc gaa cat ctg aaa 301
Asn Glu Cys Ile Ala Gln Leu Lys Asp Leu Leu Pro Glu His Leu Lys
                65                  70                  75

ttg aca aca ctg ggg cat ttg gag aaa gca gta gtc ttg gaa tta act 349
Leu Thr Thr Leu Gly His Leu Glu Lys Ala Val Val Leu Glu Leu Thr
            80                  85                  90

tta aag cac ttg aaa gcg cta aca gcc tta act gag cag cag cat cag 397
Leu Lys His Leu Lys Ala Leu Thr Ala Leu Thr Glu Gln Gln His Gln
        95                  100                 105

aag ata att gct tta cag aat ggg gag cgc tct ctg aaa tcg ccg gtc 445
Lys Ile Ile Ala Leu Gln Asn Gly Glu Arg Ser Leu Lys Ser Pro Val
    110                 115                 120

cag gcc gac ttg gat gcg ttc cac tcg ggg ttt caa acc tgc gcc aaa 493
Gln Ala Asp Leu Asp Ala Phe His Ser Gly Phe Gln Thr Cys Ala Lys
125                 130                 135                 140

gaa gtc ttg caa tac ctc gcg cgc ttt gag agc tgg aca ccc agg gag 541
Glu Val Leu Gln Tyr Leu Ala Arg Phe Glu Ser Trp Thr Pro Arg Glu
                145                 150                 155

ccg cgc tgc gca cag ctc gtc agc cac ctg cat gcc gtg gcc acc cag 589
Pro Arg Cys Ala Gln Leu Val Ser His Leu His Ala Val Ala Thr Gln
            160                 165                 170

ctc ctg acg cca cag gtg ccc tcc ggc agg ggc tct ggg cgc gcg ccc 637
Leu Leu Thr Pro Gln Val Pro Ser Gly Arg Gly Ser Gly Arg Ala Pro
        175                 180                 185

tgc agc gcg ggg gct gcg gcc gcc tcg ggt ccc gag cgc gtc gcc cgc 685
Cys Ser Ala Gly Ala Ala Ala Ala Ser Gly Pro Glu Arg Val Ala Arg

    190                 195                 200

tgc gtg ccg gtc atc cag cgg act cag ccc ggc acg gag ccg gaa cac 733
Cys Val Pro Val Ile Gln Arg Thr Gln Pro Gly Thr Glu Pro Glu His
205                 210                 215                 220

gac acg gac acc gac agc ggc tac gga ggc gag gcg gag cag ggc cgc 781
Asp Thr Asp Thr Asp Ser Gly Tyr Gly Gly Glu Ala Glu Gln Gly Arg
                225                 230                 235

gcg gcc gtc aag cag gag cca ccc ggg gac tcg tcg cct gcg ccc aag 829
Ala Ala Val Lys Gln Glu Pro Pro Gly Asp Ser Ser Pro Ala Pro Lys
            240                 245                 250

agg ccg aag ctg gag gcg cgc ggc gcg ctc ctg ggc ccg gag ccc gcg 877
Arg Pro Lys Leu Glu Ala Arg Gly Ala Leu Leu Gly Pro Glu Pro Ala
        255                 260                 265

ctg ctc ggc tcg ctc gtg gcg ctt ggc ggg ggc gcg ccc ttc gcg cag 925
Leu Leu Gly Ser Leu Val Ala Leu Gly Gly Gly Ala Pro Phe Ala Gln
    270                 275                 280

ccc gct gcc gcg ccc ttc tgc ctg ccc ttc tat ctg ctg tcg ccg tcc 973
Pro Ala Ala Ala Pro Phe Cys Leu Pro Phe Tyr Leu Leu Ser Pro Ser
285                 290                 295                 300

gcc gcc gcc tac gta cag ccc tgg cta gac aag agc ggc ctg gac aag 1021
Ala Ala Ala Tyr Val Gln Pro Trp Leu Asp Lys Ser Gly Leu Asp Lys
                305                 310                 315

tat ctg tac ccc gcg gcg gcc gcg ccc ttc ccg ctg ctg tat ccc ggc 1069
Tyr Leu Tyr Pro Ala Ala Ala Ala Pro Phe Pro Leu Leu Tyr Pro Gly
            320                 325                 330

atc cca gca gcg gcc gcc gct gct gct gcc gcc gct ttc cct tgc ttg 1117
Ile Pro Ala Ala Ala Ala Ala Ala Ala Ala Ala Ala Phe Pro Cys Leu
        335                 340                 345

tcg tcc gtg ctg tcg cca ccc ccg gag aag gcc ggc gcg acc gcc ggt 1165
Ser Ser Val Leu Ser Pro Pro Pro Glu Lys Ala Gly Ala Thr Ala Gly
    350                 355                 360

gcc ccg ttc ctg gcg cac gag gtg gcg ccc ccg ggg ccg ctg cgc ccc 1213
Ala Pro Phe Leu Ala His Glu Val Ala Pro Pro Gly Pro Leu Arg Pro
365                 370                 375                 380

cag cac gcg cat agc cgc acc cac ctg ccg cgc gct gtg aac ccg gag 1261
Gln His Ala His Ser Arg Thr His Leu Pro Arg Ala Val Asn Pro Glu
                385                 390                 395

agc tct cag gaa gat gcc acg cag ccg gcc aag gac gcc ccc 1303
Ser Ser Gln Glu Asp Ala Thr Gln Pro Ala Lys Asp Ala Pro

            400                 405                 410

tgaacccagc attccttcca gaacagggca gggggctccc gaggagtcgc cgggtttcca 1363

agttcaaacg tcctctaaag cgtgccaggg aggaagagta agcgatgctc gacaggct 1421

<210> 14

<211> 410

<212> PRT

<213> Mus musculus

<400> 14

Met Asp Glu Gly Ile Pro His Leu Gln Glu Arg Gln Leu Leu Glu His

1               5                   10                  15

Arg Asp Phe Ile Gly Leu Asp Tyr Ser Ser Leu Tyr Met Cys Lys Pro

            20                  25                  30

Lys Arg Ser Leu Lys Arg Asp Asp Thr Lys Asp Thr Tyr Lys Leu Pro

        35                  40                  45

His Arg Leu Ile Glu Lys Lys Arg Arg Asp Arg Ile Asn Glu Cys Ile

    50                  55                  60

Ala Gln Leu Lys Asp Leu Leu Pro Glu His Leu Lys Leu Thr Thr Leu

65                  70                  75                  80

Gly His Leu Glu Lys Ala Val Val Leu Glu Leu Thr Leu Lys His Leu

                85                  90                  95

Lys Ala Leu Thr Ala Leu Thr Glu Gln Gln His Gln Lys Ile Ile Ala

            100                 105                 110

Leu Gln Asn Gly Glu Arg Ser Leu Lys Ser Pro Val Gln Ala Asp Leu

        115                 120                 125

Asp Ala Phe His Ser Gly Phe Gln Thr Cys Ala Lys Glu Val Leu Gln

    130                 135                 140

Tyr Leu Ala Arg Phe Glu Ser Trp Thr Pro Arg Glu Pro Arg Cys Ala

145                 150                 155                 160

Gln Leu Val Ser His Leu His Ala Val Ala Thr Gln Leu Leu Thr Pro

                165                 170                 175

Gln Val Pro Ser Gly Arg Gly Ser Gly Arg Ala Pro Cys Ser Ala Gly

            180                 185                 190

Ala Ala Ala Ala Ser Gly Pro Glu Arg Val Ala Arg Cys Val Pro Val

        195                 200                 205

Ile Gln Arg Thr Gln Pro Gly Thr Glu Pro Glu His Asp Thr Asp Thr
    210                 215                 220

Asp Ser Gly Tyr Gly Gly Glu Ala Glu Gln Gly Arg Ala Ala Val Lys
225                 230                 235                 240

Gln Glu Pro Pro Gly Asp Ser Ser Pro Ala Pro Lys Arg Pro Lys Leu
                245                 250                 255

Glu Ala Arg Gly Ala Leu Leu Gly Pro Glu Pro Ala Leu Leu Gly Ser
            260                 265                 270

Leu Val Ala Leu Gly Gly Gly Ala Pro Phe Ala Gln Pro Ala Ala Ala
        275                 280                 285

Pro Phe Cys Leu Pro Phe Tyr Leu Leu Ser Pro Ser Ala Ala Ala Tyr
    290                 295                 300

Val Gln Pro Trp Leu Asp Lys Ser Gly Leu Asp Lys Tyr Leu Tyr Pro
305                 310                 315                 320

Ala Ala Ala Ala Pro Phe Pro Leu Leu Tyr Pro Gly Ile Pro Ala Ala
                325                 330                 335

Ala Ala Ala Ala Ala Ala Ala Ala Phe Pro Cys Leu Ser Ser Val Leu
            340                 345                 350

Ser Pro Pro Pro Glu Lys Ala Gly Ala Thr Ala Gly Ala Pro Phe Leu
        355                 360                 365
Ala His Glu Val Ala Pro Pro Gly Pro Leu Arg Pro Gln His Ala His
    370                 375                 380
Ser Arg Thr His Leu Pro Arg Ala Val Asn Pro Glu Ser Ser Gln Glu
385                 390                 395                 400
Asp Ala Thr Gln Pro Ala Lys Asp Ala Pro
                405                 410

<210> 15
<211> 21
<212> DNA
<213> Artificial Sequence

<220>
<223> Description of Artificial Sequence:Artificially
Synthesized Primer Sequence

<400> 15
aaaatctctc caggcgaccg t 21

<210> 16

<211> 20

<212> DNA

<213> Artificial Sequence

<220>

<223> Description of Artificial Sequence:Artificially
      Synthesized Primer Sequence

<400> 16

agcctgtcga gcatcgctta                                                    20

## Description

### Technical Field

The present invention relates to novel bHLH type transcription factors and genes thereof. These molecules may be used, for example in the development of pharmaceutical agents.

### Background Art

In animals, the development and differentiation are regulated by various transcription factors that control tissue differentiation and cell proliferation. Particularly, bHLH (basic domain-Helix-Loop-Helix structure) type transcription factors are reported to control cell proliferation and differentiation in myogenesis (Weintraub H. et al. (1991) Science 251:761-6), neurogenesis (Jan Y.N. and Jan L.Y. (1993) Cell 75:827-830), hematopoiesis (Zhuang Y. et al. (1994) Cell 79:875-884), and such. bHLH type transcription factors are characterized by a conserved region (bHLH structure) of 60 to 70 amino acids, which mediates dimer formation of proteins. The HLH domain is mostly located downstream adjacent to the basic (b) domain necessary for DNA binding. Generally, these proteins bind to DNA by forming homodimers or heterodimers to regulate transcription (Jan Y.N. and Jan L.Y. (1993) Cell 75:827-830). bHLH transcription factors are classified into several families according to their structural and functional characteristics (Dang C. et al. (1992) Proc.Natl.Acad.Sci.USA 89:559-602; Ohsako S. et al. (1994) Genes Dev. 8:2743-2755). Among these, HES family genes, which are mammalian homologues of the *Drosophila* hairy [h] (Rushlow C.A. et al. (1989) EMBO J. 8:3095-3103) and enhancer-of-split [E (spl)] (Klambt C. et al. (1989) EMBO J. 8:203-210) genes, are related to neurogenesis and are reported to function as repressors (negative regulatory factor) of transcription (Sasai Y. et al. (1992) Genes Dev. 6:2620-2634; Ishibashi M. et al. (1993) Eur.J.Biochem. 215:645-652; Akazawa C. et al. (1992) J.Biol.Chem. 267:21879-21885; Ohsako S. et al. (1994) Genes Dev. 8:2743-2755; Dawson S.R. et al. (1995) Mol.Cell. Biol. 15:6923-6931; Jan Y.N. and Jan L.Y. (1993) Cell 75:827-830).

The inventors previously cloned bHLH type transcription factor DEC1, the expression of which is specifically induced by dibutyryl cAMP in human cultured chondrocytes that retain the characteristics of cartilages, using the subtraction method (Unexamined Published Japanese Patent Application (JP-A) No. Hei 11-75882). DEC1 is homologous to the hairy, enhancer-of-split [E(spl)], and HES family genes(Sasai Y. et al. (1992) Genes Dev. 6:2620-2634; Ishibashi M. et al. (1993) Eur.J.Biochem. 215:645-652), and is considered to be a novel bHLH type transcription factor phylogenetically related to these transcription factors. Expression of DEC1 is observed in not only chondrocytes, but also other multiple human tissues. Furthermore, the fact that cAMP induces expression of DEC1 mRNA in many cells suggests that DEC1 is related to not only the regulation of chondrocyte differentiation, but also the regulation of differentiation and cell proliferation of other tissues (Shen M. et. al. (1997) Biochem.Biophys.Res.Commun. 236:294-298).

Besides DEC1, mouse Stra13 (Boudjelal M. et al. (1997) Genes Dev. 11:2052-2065) and rat SHARP (Rossner M.J. et al. (1997) Mol.Cell.Neurosci. 9:460-475) are also identified as bHLH type transcription factors that show relatively high homology with h/E(spl)/HES. The expression of mouse Stra13 is induced when embryonal carcinoma cell line P19 is treated with retinoic acid, and a high expression induces differentiation of neurocytes. Moreover, this transcription factor represses transcriptional activation of other transcription factors by forming a heterodimer, functioning as a transcriptional repressor. During embryogenesis, expression of Stra13 is observed in the endoderm and mesoderm, besides the neuroectoderm (Boudjelal M. et al. (1997) Genes Dev. 11:2052-2065). Rat SHARP is a bHLH type transcription factor whose expression rises in the late phase of embryogenesis and after birth and is expected to function in the plasticity of the brain. Furthermore, its expression is induced by the addition of NGF to PC12 cells, or by the addition of kainic acid *in vivo.* Two genes, SHARP-2 and SHARP-1, showing different tissue distributions have been identified as rat SHARP genes. Therefore, it is supposed that human DEC1 also forms a subfamily with other analogous genes. The isolation of these unknown bHLH type transcription factors is thought to be an important step in developing new pharmaceutical agents.

### Disclosure of the Invention

An objective of the present invention is to provide novel bHLH type transcription factors and genes thereof, as well as methods for producing them, and their uses.

The present inventors searched for human cDNA fragments that are similar to DEC1 in an EST database to clone a novel transcription factor that constitutes a DEC1 subfamily. Using a primer synthesized based on the nucleotide sequence information of a thus obtained cDNA fragment, PCR cloning was conducted using human skeletal muscle cDNA as template. Furthermore, 3'RACE and 5'RACE methods were performed to obtain multiple cDNA, and the full-length cDNA sequences were determined. One of these cDNA with a full-length of 3641 bp encoded a protein of 482 amino acids , the molecular weight of which was estimated to be 50.5 kDa (this clone was named "human DEC2a"). The amino acid sequence of human DEC2a showed the highest similarity to that of rat SHARP-1. Particularly, the N-terminus half was 90% or more identical, whereas the C-terminus half showed a low similarity where only parts of the sequence were identical. This protein was supposed to be a new member of the DEC1 subfamily, because the bHLH region showed a high similarity. Another cDNA with a full-length of 1511 bp encoded a protein of 484 amino acids and was presumed to have a molecular weight of 50.7 kDa. This clone showed significant homology with human DEC2a and thus was named "human DEC2b".

Moreover, the present inventors succeeded in obtaining the mouse DEC2a cDNA corresponding to the human DEC2a cDNA, which has a full-length of 1421 bp and encodes a protein of 410 amino acids having an estimated molecular weight of 43.9 kDa, by PCR using mouse skeletal muscle cDNA as the template.

The novel bHLH type transcription factors "DEC2" isolated by the present inventors are thought to be members of the DEC1 subfamily. Consequently, they are expected to be useful as novel factors that control development and tissue differentiation. It is also expected that they may be used as markers to determine developmental stages and cell differentiation. Additionally, they are expected to be useful as targets in developing pharmaceutical agents for various diseases associated with the proteins of the present invention.

The present invention relates to novel bHLH type transcription factors and their genes, their production, and uses as well. More specifically, it provides:
(1) a DNA according to any one of the following (a) to (d):
   (a) a DNA comprising the coding region of the nucleotide sequence of SEQ ID NO: 1, 11, or 13,
   (b) a DNA encoding a protein comprising the amino acid sequence of SEQ ID NO: 2, 12, or 14,
   (c) a DNA encoding a protein comprising an amino acid sequence in which one or more amino acids in the amino acid sequence of SEQ ID NO: 2, 12, or 14 has been substituted, deleted, inserted, and/or added, wherein the protein is functionally equivalent to the protein comprising the amino acid sequence of SEQ ID NO: 2, 12, or 14, and,
   (d) a DNA that hybridizes under stringent conditions to a DNA comprising the nucleotide sequence of SEQ ID NO: 1, 11, or 13, and encodes a protein that is functionally equivalent to the protein comprising the amino acid sequence of SEQ ID NO: 2, 12, or 14;
(2) a DNA that encodes a partial peptide of the protein of SEQ ID NO: 2, 12, or 14;
(3) a vector into which a DNA of (1) or (2) is inserted;
(4) a transformant carrying a DNA of (1) or (2), or the vector of (3) ;
(5) a protein or peptide encoded by a DNA of (1) or (2);
(6) a method for producing the protein or peptide of (5) , comprising the steps of:
   culturing the transformant of (4), and,
   recovering the expressed protein from the transformant or culture supernatant;
(7) a DNA comprising at least 15 nucleotides, wherein the DNA is complementary to a DNA comprising the nucleotide sequence of SEQ ID NO: 1, 11, or 13, or to the complementary strand thereof;
(8) a method of screening for a compound that binds to the protein of (5), comprising the steps of:
   (a) contacting a test sample with said protein or partial peptide,
   (b) detecting the binding activity of said protein or partial peptide with the test sample, and,
   (c) selecting a compound that has a binding activity to said protein or partial peptide;
(9) a compound that binds to the protein of (5);
(10) the compound of (9), wherein said compound is an antibody; and
(11) the compound of (9), wherein said compound is isolated by a method as set forth in (8).

The present invention provides novel bHLH type transcription factors and DNA encoding the proteins. The nucleotide sequences of the cDNA of bHLH type transcription factors, human DEC2a, human DEC2b, and mouse DEC2a (collectively called "DEC2") isolated by the present inventors, and the amino acid sequences of the proteins encoded by the cDNA are shown in SEQ ID NO: 1, 11, 13, 2, 12, and 14, respectively. The "DEC2" genes isolated by the present inventors are suggested to be related not only to differentiation and proliferation of tissues including cartilages, but also to functions of various adult tissues since they were highly homologous to bHLH type transcription factor SHARP in the bHLH region , which is suggested to be involved in the plasticity of cells of the central nervous system in rats. Thus , "DEC2" proteins of the present invention and DNA encoding these proteins are not only useful as factors that control tissue differentiation and cell function, or as differentiation markers, but they may also be applied to the diagnosis, prophylaxis, and treatment of diseases related to the proteins of the present invention.

For example, "DEC2" is important in elucidating the differentiation and deformation mechanisms of cartilages, and it is also expected to be useful in developing gene therapy methods against osteoarthritis, rheumatoid arthritis, etc.

Furthermore, the present invention includes proteins functionally equivalent to the "DEC2" proteins (SEQ ID NOs: 2, 12, and 14). Such proteins include, for example, homologue proteins derived from other organisms , which correspond to the "DEC2" proteins , and mutants of "DEC2" proteins. Herein, "functionally equivalent" means that the target protein has a function of a bHLH type transcription factor. The function of a bHLH type transcription factor is to form a homodimer (or a heterodimer with other bHLH type transcription factors) and have an activity to negatively or positively regulate transcriptional activity. Further, a bHLH type transcription factor may have a binding activity towards CANNTG and/or CACNAG. Methods for measuring the binding activity towards CANNTG and/or CACNAG are well known (for example, Ohsako et al. (1994) Genes&Dev. 8:2743-2755).

One method well known to those skilled in the art for preparing functionally equivalent proteins is to introduce mutations into proteins. For example, one skilled in the art can prepare proteins functionally equivalent to a "DEC2" protein by introducing appropriate mutations into the amino acid sequence of the protein (SEQ ID NO: 2, 12, or 14), by using site-specific mutagenesis (Hashimoto-Gotoh, T. et al. (1995) Gene 152, 271-275; Zoller, MJ, and Smith, M. (1983) Methods Enzymol. 100, 468-500; Kramer, W. et al. (1984) Nucleic Acids Res. 12, 9441-9456; Kramer W, and Fritz HJ(1987) Methods. Enzymol. 154, 350-367; Kunkel, TA(1985) Proc Natl Acad Sci U S A. 82, 488-492; Kunkel (1988) Methods Enzymol. 85, 2763-2766), and such. Mutation of amino acids may occur in nature, too. Furthermore, the proteins of the present invention include a protein comprising an amino acid sequence of a "DEC2" protein (SEQ ID NO: 2, 12, or 14) in which one or more amino acids have been mutated, which is functionally equivalent to the DEC2 protein. In such a mutant protein, the number of amino acids mutated are considered to be usually 100 residues or less, preferably 50 residues or less, more preferably 20 residues or less, even more preferably 10 residues or less, and still more preferably 5 residues or less.

It is preferable to mutate an amino acid residue into one that allows the properties of the amino acid side-chain to be conserved. Examples of properties of amino acid side chains include: hydrophobic amino acids (A, I, L, M, F, P, W, Y, V), hydrophilic amino acids (R, D, N, C, E, Q, G, H, K, S, T), and amino acids comprising the following side chains: aliphatic side-chains (G, A, V, L, I, P); hydroxyl group-containing side-chains (S, T, Y); sulfur atom-containing side-chains (C, M) ; carboxylic acid- and amide-containing side-chains (D, N, E, Q); base-containing side-chain (R, K, H); and aromatic-containing side-chains (H, F, Y, W) (The letters within parenthesis indicate the one-letter codes of amino acids).

It is well known that a protein having a deletion, addition, and/or substitution of one or more amino acid residues in the sequence of the protein can retain the original biological activity (Mark D.F. et al. Proc. Natl. Acad. Sci. U.S.A. 81:5662-5666 (1984); Zoller M.J. and Smith M. Nucleic Acids Res. 10:6487-6500 (1982); Wang A. et al. Science 224:1431-1433; Dalbadie-McFarland G. et al. Proc. Natl. Acad. Sci. U.S.A. 79:6409-6413 (1982)).

A protein in which amino acid residues have been added to the amino acid sequence of a "DEC2" protein includes a fusion protein comprising the "DEC2" protein. The present invention includes a fusion protein in which one or more "DEC2" proteins and one or more other proteins or peptides are fused. Methods well known in the art may be used to generate a fusion protein of the present invention. For example, a DNA encoding a "DEC2" protein (SEQ ID NO: 2, 12, or 14) and a DNA encoding another protein or peptide are linked in frame and introduced into an expression vector. The fusion protein is then expressed in a host cell. The protein or peptide fused to a protein of the present invention is not limited to any specific protein or peptide.

Known peptides, for example, FLAG (Hopp, T.P. et al., Biotechnology (1988) 6, 1204-1210), 6xHis containing six His (histidine) residues, 10xHis, Influenza agglutinin (HA), human c-myc fragment, VSP-GP fragment, p18HIV fragment, T7-tag, HSV-tag, E-tag, SV40T antigen fragment, l ck tag, α-tubulin fragment, B-tag, Protein C fragment, and such, can be used as peptides that are fused to a protein of the present invention. Examples of proteins that are fused to a protein of the invention are, GST (glutathione-S-transferase), Influenza agglutinin (HA), immunoglobulin constant region, β-galactosidase, MBP (maltose-binding protein), and such.

Fusion proteins can be prepared by fusing commercially available DNA encoding these peptides or proteins with a DNA encoding a protein of the present invention and expressing the fused DNA prepared.

An alternative method known to those skilled in the art for preparing functionally equivalent proteins is, for example, the method utilizing the hybridization technique (Sambrook, J. et al., Molecular Cloning 2nd ed. 9.47-9.58, Cold Spring Harbor Lab. Press, 1989). Generally, one skilled in the art can isolate DNA highly homologous to the whole or part of a DNA sequence encoding a "DEC2" protein (SEQ ID NO: 1, 11, or 13), and then isolate a protein functionally equivalent to the "DEC2" protein from those DNA isolated. The proteins of the present invention thus include proteins encoded by DNA that hybridize with the whole or part of a DNA sequence encoding a "DEC2" protein under stringent conditions, in which the proteins are functionally equivalent to the "DEC2" protein. These proteins include mammalian homologues (e.g. proteins encoded by genes of humans, monkeys, mice, rats, rabbits, cattle, pigs, dogs, and cats) . In isolating cDNA highly homologous to a DNA encoding a "DEC2" protein from animals, it may be preferable to use tissues such as brain, skeletal muscle, testis, placenta, large intestine, spleen, cartilage, and so on.

Hybridization conditions for isolating a DNA encoding a protein functionally equivalent to a "DEC2" protein may be appropriately selected by a person skilled in the art. A hybridization conducted under stringent conditions is one conducted, for example, in a solution of 6x SSC (0.9 M sodium chloride, 0.09 M sodium citrate), 0.5% SDS, 10 mM EDTA, 5x Denhardt's solution (0.1%(w/v) Ficoll, 0.1% (w/v) polyvinylpyrrolidone) , 0.1% (w/v) BSA), 10 mg/ml denatured salmon sperm DNA at 60°C. A more preferable stringent condition is conducting hybridization at 68°C in the solution above. However, other than temperature, several factors, such as salt concentration, can influence the stringency of hybridization and one skilled in the art can suitably select the factors to accomplish a similar stringency.

In place of hybridization, a gene amplification method using primers synthesized based on the sequence information of the DNA (SEQ ID NO: 1, 11, or 13) encoding the "DEC2" proteins, for example, the polymerase chain reaction (PCR) method, can be utilized.

A protein functionally equivalent to a "DEC2" protein encoded by the DNA isolated through the above hybridization technique or gene amplification techniques normally has a high homology to the amino acid sequence of a "DEC2" protein (SEQ IDNO: 2, 12, or 14) . The proteins of the present invention also include proteins that are functionally equivalent to a "DEC2" protein and are highly homologous to the amino acid sequence shown in SEQ ID NO: 2, 12, or 14. "Highly homologous" refers to, normally an identity of 60% or higher, preferably 70% or higher, more preferably 80% or higher. The homology of a protein can be determined by following the algorithm in "Wilbur, W.J. and Lipman, D.J. (1983) Proc. Natl. Acad. Sci. USA 80:726-730".

The proteins of the present invention may have variations in the amino acid sequence, molecular weight, isoelectric point, the presence or absence of sugar chains, form, and so on, depending on the cell or host used to produce it or the purification method utilized (described below). Nevertheless, as long as the obtained protein has a function equivalent to a "DEC2" protein, it is within the scope of the present invention. For example, if a protein of the present invention is expressed in a prokaryotic cell such as *E.coli,* the protein includes a methionine residue at the N-terminus in addition to the natural amino acid sequence of the protein. Such proteins are also included in the proteins of the present invention.

The proteins of the present invention can be prepared recombinant proteins or naturally occurring proteins, using methods commonly known intheart. When the protein is a recombinantprotein, it may be produced by inserting a DNA (for example, a DNA having the nucleotide sequence of SEQ ID NO: 1, 11, or 13) encoding a protein of the present invention into an appropriate expression vector, collecting the transformant obtained by introducing the vector into an appropriate host cell, obtaining an extract, and then purifying and preparing the protein using ion exchange, reverse phase, gel filtration, or affinity chromatography. Affinity chromatography may be done using a column in which an antibody against a protein of the present invention is fixed. A combination of such columns may also be used.

Alternatively, when a protein of the invention is expressed in host cells (e.g., animal cells or *E. coli)* as a fusion protein with glutathione S transferase protein, or a recombinant protein with multiple histidine residues, the expressed recombinant protein can be purified using a glutathione column or nickel column.

After the fusion protein is purified, if necessary, regions of the fusion protein (apart from the desired protein) can be digested and removed with thrombin, factor Xa, etc.

The native protein of the invention can be isolated by methods well known in the art, for example, purifying an extract of tissues or cells that express a protein of the invention with an affinity column to which an antibody binding to a protein of the present invention described below is bound. The antibody may be a polyclonal or monoclonal antibody.

The present invention also includes partial peptides of the proteins of the present invention. The partial peptides of the present invention comprise at least 8 or more amino acids, preferably 15 or more amino acids, more preferably 30 or more amino acids, and still more preferably 50 or more amino acids (for example, 100 amino acids ormore) . The partial peptides can be used, for example, for generating antibodies against a protein of the present invention, screening of compounds binding to a protein of the present invention, or screening of stimulators or inhibitors of a protein of the present invention. Additionally, they may be antagonists or competitive inhibitors of the proteins of the present invention. The partial peptides of the proteins of the present invention include those that include, for example, the bHLH region of a protein comprising the amino acid sequence shown in SEQ ID NO: 2, 12, or 14.

The partial peptides of the present invention can be produced by genetic engineering methods, known peptide synthesis methods, or by cutting the proteins of the present invention by appropriate peptidases. Synthesis of the peptides may be conducted according to, for example, the solid phase synthesis method, or the liquid phase synthesis method.

In addition to being utilized in the above-described *in vivo* or *in vitro* production of a protein of the present invention, a DNA encoding a protein of the present invention may also be applied, for example, in the gene therapy of diseases caused by an aberration in a gene encoding a protein of the present invention or diseases treatable by a protein of the present invention. Any type of DNA, such as cDNA synthesized from mRNA, genomic DNA, or synthetic DNA can be used so long as the DNA encodes a protein of the present invention. Also as long as they can encode a protein of the present invention, DNA comprising arbitrary sequences based on the degeneracy of the genetic code are also included.

The DNA of the present invention can be prepared by using methods known in the art. For example, a cDNA library can be constructed from cells expressing a protein of the present invention and hybridization can be conducted using a part of the DNA sequence of the present invention (for example, SEQ ID NO: 1, 11, or 13) as a probe. The cDNA library may be prepared, for example, according to the method described by Sambrook J. et al. (Molecular Cloning, Cold Spring Harbor Laboratory Press (1989)), or instead, commercially available cDNA libraries may be used. Alternatively, a DNA of the present invention can be obtained by preparing RNA from cells expressing a protein of the present invention, synthesizing cDNA by using a reverse transcriptase, synthesizing oligo-DNA based on a DNA sequence of the present invention (for example, SEQ ID NO: 1, 11, or 13), and amplifying the cDNA encoding a protein of the present invention by PCR using the oligo-DNA as primers.

The nucleotide sequence of the obtained cDNA is determined to find an open reading frame, and thereby, the amino acid sequence of a protein of the invention can be obtained. The cDNA obtained may also be used as a probe for screening a genomic library to isolate genomic DNA.

More specifically, mRNA may first be isolated from a cell, tissue, or organ in which a protein of the invention is expressed (e.g. tissues such as brain, skeletal muscle, testis, placenta, large intestine, spleen, and cartilage). Known methods can be used to isolate mRNA; for instance, total RNA is prepared by guanidine ultracentrifugation (Chirgwin J.M. et al. Biochemistry 18:5294-5299 (1979)) or AGPC method (Chomczynski P. and Sacchi N. Anal. Biochem. 162:156-159 (1987)), and mRNA is purified from total RNA using an mRNA Purification Kit (Pharmacia) and such. Alternatively, mRNA may be directly prepared by QuickPrep mRNA Purification Kit (Pharmacia).

The obtained mRNA is used to synthesize cDNA using reverse transcriptase. cDNA may be synthesized by using a kit such as the AMV Reverse Transcriptase First-strand cDNA Synthesis Kit (Seikagaku Kogyo). Alternatively, cDNA may be synthesized and amplified following the 5'-RACE method (Frohman M.A. et al. Proc. Natl. Acad. Sci. U.S.A. 85:8998-9002 (1988); Belyavsky A. et al. Nucleic Acids Res. 17:2919-2932 (1989)) that uses primers and such described herein, the 5'-Ampli FINDER RACE Kit (Clontech), and polymerase chain reaction (PCR).

A desired DNA fragment is prepared from the PCR products and linked to a vector DNA. The recombinant vector is used to transform *E. coli* and such, and the desired recombinant vector is prepared from a selected colony. The nucleotide sequence of the desired DNA can be verified by conventional methods, such as dideoxynucleotide chain termination.

A DNA of the invention may be designed to have a sequence that is expressed more efficiently by taking into account the frequency of codon usage in the host used for expression (Grantham R. et al. Nucleic Acids Res. 9:43-74 (1981)). The DNA of the present invention may be altered by a commercially available kit or a conventional method. For instance, the DNA may be altered by digestion with restriction enzymes, insertion of a synthetic oligonucleotide or an appropriate DNA fragment, addition of a linker, or insertion of the initiation codon (ATG) and/or the stop codon (TAA, TGA, or TAG), etc.

Specifically, the DNA of the present invention include DNA having the following nucleotide sequences: from A at position 135 to C at position 1580 of SEQ ID NO: 1, from A at position 2 to C at position 1453 of SEQ ID NO: 11, and from A at position 74 to C at position 1303 of SEQ ID NO: 13.

Furthermore, the DNA of the present invention include DNA capable of hybridizing with DNA having the nucleotide sequence of SEQ ID NO: 1, 11, or 13 under stringent conditions, and encoding a protein functionally equivalent to a protein of the invention described above. Stringent hybridization conditions may be appropriately chosen by one skilled in the art. Specifically, conditions described above may be used. Under the conditions, DNA having higher homologies can be obtained by increasing temperature. The above hybridizing DNA is preferably a natural DNA, for example, a cDNA or a chromosomal DNA.

The present invention also provides a vector into which a DNA of the present invention is inserted. The vectors of the present invention are useful in maintaining the DNA of the present invention within the host cell, or expressing a protein of the present invention.

When *E. coli* is used as the host cell, there is no limitation other than that the vector should have an "ori", to amplify and mass-produce the vector in *E. coli* (e.g., JM109, DH5α, HB101, or XL1Blue), and such, and a marker gene for selecting the transformed *E. coli* (e.g., a drug-resistance gene selected by a drug (e.g., ampicillin, tetracycline, kanamycin, or chloramphenicol)). For example, M13-series vectors, pUC-series vectors, pBR322, pBluescript, pCR-Script, and such can be used. Besides the vectors , pGEM-T, pDIRECT, pT7, and so on can also be used for the subcloning and excision of the cDNA as well. When a vector is used to produce a protein of the present invention, an expression vector is especially useful. When the expression vector is expressed, for example, in *E. coli,* it should have the above characteristics in order to be amplified in *E. coli.* Additionally, when *E*. *coli,* such as JM109, DH5α, HB101, or XL1-Blue, are used as the host cell, the vector should have a promoter, e.g. lacZ promoter (Ward et al. (1989) Nature 341:544-546; (1992) FASEB J. 6:2422-2427), araB promoter (Better et al. (1988) Science 240 :1041-1043), or T7 promoter, that can efficiently promote the expression of the desired gene in *E.coli*. Other examples of the vectors are pGEX-5X-1 (Pharmacia), "QIAexpress system" (QIAGEN) , pEGFP, and pET (for this vector, BL21, a strain expressing T7 RNA polymerase, is preferably used as the host).

Further, the vector may comprise a signal sequence to secrete the polypeptide. For producing the protein into the periplasm of *E*. *coli*, the pelB signal sequence (Lei S.P. et al. *J.* Bacteriol. 169:4379 (1987)) may be used as the signal sequence for protein secretion. For example, the calcium chloride method or electroporation may be used to introduce the vector into host cells.

As vectors used to produce the proteins of the present invention, for example, expression vectors derived from mammals (e.g. pCDNA3 (Invitrogen) , pEGF-BOS (Nucleic Acids Res. (1990) 18 (17) :5322) , pEF, pCDM8) , insect cells (e.g. "Bac-to-BAC baculovirus expression system" (GIBCO-BRL) , pBacPAK8), plants (e.g. pMH1, pMH2), animal viruses (e.g. pHSV, pMV, pAdexLcw), retroviruses (e.g. pZIPneo), yeasts (e.g. "Pichia Expression Kit" (Invitrogen), pNV11, SP-Q01), and *Bacillus subtilis* (e.g. pPL608, pKTH50) can be mentioned other than *E.coli.*

In order to express proteins in animal cells, such as CHO, COS, and NIH3T3 cells, the vector must have a promoter necessary for expression in such cells (e.g. SV40 promoter (Mulligan et al. (1979) Nature 277:108), MMLV-LTR promoter, EF1α promoter (Mizushima et al. (1990) Nucleic Acids Res. 18:5322), CMV promoter, etc.). It is more preferable if the vector additionally had a marker gene for selecting transformants (for example, a drug resistance gene selected by a drug (e.g., neomycin, G418, etc.)). Examples of vectors with such characteristics include pMAM, pDR2, pBK-RSV, pBK-CMV, pOPRSV, pOP13, and so on.

Furthermore, in order to stably express the gene and to amplify the copy number in cells, the method using CHO cells deficient in nucleic acid synthetic pathways as the host, incorporating into the CHO cells a vector (such as pCHOI) having a DHFR gene that compensates for the deficiency, and amplifying the vector with methotrexate (MTX) can be used. Furthermore, for transiently expressing a gene, the method that transforms COS cells that have the gene for SV40 T antigen on the chromosome with a vector (such as pcD) having the SV40 replication origin can be mentioned. The replication origin may be that of a polyomavirus, adenovirus, bovine papilloma virus (BPV) , and the like. Further, to amplify the gene copy number in the host cells, selection markers such as the aminoglycoside transferase (APH) gene, thymidine kinase (TK) gene, *E.coli* xanthine-guanine phosphoribosyl transferase (Ecogpt) gene, and the dihydrofolate reductase (dhfr) gene may be comprised in the expression vector.

A DNA of the present invention can be expressed in animals by, for example, inserting a DNA of the invention into an appropriate vector and introducing the vector into a living body by the retrovirus method, liposome method, cationic liposome method, adenovirus method, and so on. Thus, it is possible to perform gene therapy of diseases caused by a mutation of the "DEC2" gene of the present invention. The vectors used in these methods include, but are not limited to, adenovirus vectors (e.g. pAdex1cw), retrovirus vectors (e.g. pZIPneo), and so on. General techniques for gene manipulation, such as insertion of the DNA of the invention into a vector, can be performed according to conventional methods (Molecular Cloning, 5.61-5.63). Administration to the living body may be performed according the *ex vivo* method or the *in vivo* method.

The present invention also provides a host cell into which a vector of the present invention has been introduced. The host cell into which the vector of the invention is introduced is not particularly limited. For example, *E.coli,* various animal cells, and such, can be used. The host cell of the present invention can be used, for example, as a production system to produce and express a protein of the present invention. Protein production systems include *in vitro* and *in vivo* systems. Such production systems using eukaryotic cells or prokaryotic cells can be given as in vitro production systems.

As eukaryotic host cells, for example, animal cells, plant cells, and fungi cells can be used. Mammalian cells, for example, CHO (J.Exp.Med. (1995) 108:945), COS, 3T3, myeloma, BHK (baby hamster kidney), HeLa, Vero, amphibian cells (e.g. platanna oocytes (Valle et al. (1981) Nature 291:358-340), and insect cells (e.g. Sf9, Sf21, Tn5) are known as animal cells. Among CHO cells, those deficient in the DHFR gene, dhfr-CHO (Proc.Natl.Acad.Sci.USA-(1980) 77:4216-4220) and CHO K-1 (Proc.Natl.Acad.Sci.USA (1968) 60:1275), are particularly preferable. Among animal cells, CHO cells are particularly preferable for mass expression. A vector can be introduced into a host cell by, for example, the calcium phosphate method, the DEAE-dextran method, methods using cationic liposome DOTAP (Boehringer-Mannheim), electroporation, lipofection, etc.

As plant cells, for example, plant cells originating from *Nicotiana tabacum* are known as protein producing systems and may be used as callus cultures. As fungal cells, yeast cells such as Saccharomyces, including *Saccharomyces cerevisiae*, or filamentous fungi such as *Aspergillus,* including *Aspergillus niger,* are known.

Useful prokaryotic cells include bacterial cells. Bacterial cells such as *E.coli,* for example, JM109, DH5α, HB101, and such, as well as *Bacillus subtilis* are known.

These cells are transformed by a desired DNA, and the resulting transformants are cultured *in vitro* to obtain the protein. Transformants can be cultured using known methods. For example, culture medium such as DMEM, MEM, RPMI1640, or IMDM may be used with or without serum supplements such as fetal calf serum (FCS) as culture medium for animal cells. The pH of the culture medium is preferably between about 6 and 8. Such cells are typically cultured at about 30 to 40°C for about 15 to 200 hr, and the culture medium may be replaced, aerated, or stirred if necessary.

Animal and plant hosts may be used for *in vivo* production. For example, a desired DNA can be introduced into an animal or plant host. Encoded proteins are produced *in vivo*, and then recovered. These animal and plant hosts are included in the "host" of the present invention.

Animals to be used for the production system described above include mammals and insects. Mammals such as goats, pigs, sheep, mice, and cattle may be used (Vicki Glaser, SPECTRUM Biotechnology Applications (1993)). Alternatively, the mammals may be transgenic animals.

For instance, a desired DNA may be prepared as a fusion gene with a gene such as goat β casein gene that encodes a protein specifically produced into milk. DNA fragments comprising the fusion gene are injected into goat embryos, which are then introduced back to female goats. Proteins are recovered from milk produced by the transgenic goats (i.e., those born from the goats that had received the modified embryos) or from their offspring. To increase the amount of milk containing the proteins produced by transgenic goats, appropriate hormones may be administered (Ebert K.M. et al. (1994) Bio/Technology 12:699-702).

Alternatively, insects, such as the silkworm, may be used. Baculoviruses into which a DNA encoding a desired protein has been inserted can be used to infect silkworms, and the desired protein is recovered from the body fluid (Susumu M. et al. (1985) Nature 315:592-594)

As plants, for example, tobacco can be used. When using tobacco, a DNA encoding a desired protein may be inserted into a plant expression vector, such as pMON 530, which is introduced into bacteria, such as *Agrobacterium tumefaciens*. Then, the bacteria is used to infect tobacco, such as *Nicotiana tabacum*, and the desired polypeptide is recovered from the leaves (Julian K.-C. Ma et al. (1994) Eur. J. Immunol. 24:131-138).

A protein of the present invention obtained as above may be isolated from inside or outside of hosts (medium, etc.) , and purified as a substantially pure homogeneous protein. The method for protein isolation and purification is not limited to any specific method; in fact, any standard method may be used. For instance, column chromatography, filters, ultrafiltration, salting out, solvent precipitation, solvent extraction, distillation, immunoprecipitation, SDS-polyacrylamide gel electrophoresis, isoelectric point electrophoresis, dialysis, and recrystallization may be appropriately selected and combined to isolate and purify the protein.

For chromatography, for example, affinity chromatography, ion-exchange chromatography, hydrophobic chromatography, gel filtration chromatography, reverse phase chromatography, adsorption chromatography, and such may be used (Strategies for Protein Purification and Characterization: A Laboratory Course Manual. Ed. Daniel R. Marshak et al., Cold Spring Harbor Laboratory Press (1996)). These chromatographies may be performed by liquid chromatographies such as HPLC and FPLC. Thus, the present invention provides highly purified proteins produced by the above methods.

A protein may be optionally modified or partially deleted by treating it with an appropriate protein-modifying enzyme before or after purification. For example, trypsin, chymotrypsin, lysylendopeptidase, protein kinase, glucosidase, and such are used as protein-modifying enzymes.

The present invention also provides antibodies binding to a protein of the present invention. The antibodies of the present invention may take any form, including monoclonal antibodies, as well as polyclonal antibodies. Furthermore, antiserum obtained by immunizing animals such as rabbits and the like with a protein of the invention, all classes of polyclonal and monoclonal antibodies, as well as human and humanized antibodies produced by genetic recombination are included.

A protein of the invention used as a sensitizing antigen to obtain antibodies may be derived from any animal species, but preferably it is from a mammal such as human, mouse, or rat, and more preferably from a human. A human-derived protein may be obtained by using a nucleotide or amino acid sequence disclosed herein.

A full-length protein or a partial peptide thereof may be used as a sensitized antigen in the present invention. A partial peptide may be, for example, an amino (N)-terminus or carboxy (C)-terminus fragment of the protein. Herein, an "antibody" is defined as an antibody that reacts with either the full length or a fragment of the protein.

A gene encoding a protein of the invention or its fragment may be inserted into a known expression vector used to transform a host cell as described herein. The desired protein or its fragment may be recovered from the outside or inside of host cells by any standard method, and may be used as the sensitized antigen. Alternatively, cells expressing the protein or their lysates, or a chemically synthesized protein may be used as an antigen. Preferably, short peptides are used as antigens by appropriately binding to carrier proteins such as keyhole limpet hemocyanin, bovine serum albumin, and ovalbumin.

Any mammal may be immunized with the sensitized antigen, but preferably, the compatibility with parental cells used for cell fusion is taken into account. In general, animals of Rodentia, Lagomorpha, or Primates are used.

Animals of Rodentia include, for example, mice, rats, and hamsters. Animals of Lagomorpha include, for example, rabbits. Animals of Primates include, for example, monkeys of Catarrhini (old world monkeys) such as *Macaca fascicularis*, rhesus monkeys, sacred baboons, or chimpanzees.

Methods for immunizing animals with antigens are well known. Intraperitoneal injection or subcutaneous injection of antigens is used as a standard method. More specifically, antigens may be diluted and suspended in an appropriate amount with phosphate buffered saline (PBS), physiological saline, etc. If desired, the antigen suspension may be mixed with an appropriate amount of a standard adjuvant, such as Freund's complete adjuvant, made into an emulsion, and then administered to mammals. Preferably, this is followed by several administrations of the antigen mixed with an appropriate amount of Freund's incomplete adjuvant every 4 to 21 days. An appropriate carrier may also be used for immunization. After the above immunization, the serum is examined for an increase of the amount of desired antibodies by a standard method.

Polyclonal antibodies raised against a protein of the present invention may be prepared by collecting blood from the immunized mammal after confirming the increase of desired antibodies in the serum, and by separating serum from the blood by any conventional method. Serum containing a polyclonal antibody may also be used as a polyclonal antibody, or if necessary, the fraction containing the polyclonal antibody may be isolated from the serum. For example, fractions that recognize only a protein of the present invention are obtained by using affinity columns to which the present protein is coupled, and by further purifying the fraction using a protein A or G column, immunoglobulin G or M may be prepared.

To prepare monoclonal antibodies, immune cells are collected from a mammal immunized with an antigen and checked for an increase of the level of the desired antibodies in the serum as described above, and these cells are subjected to cell fusion. The immune cells used for cell fusion are preferably obtained from the spleen. The other parent cell fused with the above immune cell is preferably a mammalian myeloma cell, and more preferably, a myeloma cell that has acquired a special feature that can be used for selecting fusion cells by a drug.

The above immune cell and myeloma cell may be fused by basically any standard method, such as those described in literature (Galfre G. and Milstein C. Methods Enzymol. 73:3-46 (1981)).

Resulting hybridomas obtained by the cell fusion may be selected by cultivating them in a standard selection medium, such as the HAT medium (hypoxanthine, aminopterin, and thymidine containing medium) The cell culture is typically continued in the HAT medium for a period of time that is sufficient to allow all cells except the desired hybridoma (non-fused cells) to die, usually from several days to several weeks. Then, standard limiting dilution is performed to screen and clone a hybridoma cell producing the desired antibody.

Besides the above method in which a nonhuman animal is immunized with an antigen for preparing a hybridoma, human lymphocytes such as those infected by the EB virus may be immunized with a protein, protein-expressing cells, or their lysates *in vitro.* Then, the immunized lymphocytes are fused with human-derived myeloma cells that are capable of indefinite division, such as U266, to yield a hybridoma producing a desired human antibody capable of binding to a protein of the invention (JP-A No. Sho 63-17688).

Subsequently, the hybridomas thus obtained are transplanted into the abdominal cavity of a mouse from which the ascites is collected. The monoclonal antibodies thus obtained can be purified by, for example, ammonium sulfate precipitation or column chromatography using a protein A or protein G column, a DEAE ion exchange column, an affinity column and such to which a protein of the invention is coupled. An antibody of the invention can be used not only for purifying and detecting a protein of the invention, but also as a candidate for an agonist or antagonist of a protein of the present invention. Such an antibody is also expected to be used for antibody therapy of diseases in which the proteins of this invention are involved. To administer the obtained antibody to human bodies (namely, antibody therapy), human antibodies or humanized antibodies are preferred to reduce immunogenicity.

For example, transgenic animals having a repertory of human antibody genes may be immunized with a protein, protein expressing cells, or their lysates as antigen. Antibody producing cells are collected from the animals, and fused with myeloma cells to obtain hybridoma, from which human antibodies against the protein can be prepared (see WO92-03918, WO93-2227, WO94-02602, WO94-25585, WO96-33735, and WO96-34096).

Alternatively, an immune cell that produces antibodies, such as an immunized lymphocyte, may be immortalized by an oncogene and used for preparing monoclonal antibodies.

Such monoclonal antibodies can also be recombinantly prepared using genetic engineering techniques (see, for example, Borrebaeck C.A.K. and Larrick J.W. Therapeutic Monoclonal Antibodies, published in the United Kingdom by MacMillan Publishers LTD (1990)). A recombinant antibody can be prepared by cloning a DNA encoding the antibody from an immune cell such as a hybridoma or an immunized lymphocyte producing the antibody, inserting this into an appropriate vector, and introducing the vector into an host cell. The present invention also encompasses recombinant antibodies prepared as described above.

An antibody of the present invention may be a fragment of an antibody or modified antibody, so long as it binds to one or more of the proteins of the invention. For instance, the antibody fragment may be Fab, F(ab')₂, Fv, or single chain Fv (scFv), in which Fv fragments from H and L chains are linked by an appropriate linker (Huston J.S. et al. (1988) Proc. Natl. Acad. Sci. U.S.A. 85:5879-5883). More specifically, an antibody fragment may be generated by treating an antibody with an enzyme such as papain or pepsin. Alternatively, a gene encoding the antibody fragment may be constructed, inserted into an expression vector, and expressed in an appropriate host cell (see, for example, Co M.S. et al. (1994) J. Immunol. 152:2968-2976; Better M. and Horwitz A.H. (1989) Methods Enzymol. 178:476-496; Pluckthun A. and Skerra A. (1989) Methods Enzymol. 178:497-515 ; Lamoyi E. Methods Enzymol. (1986) 121:652-663; Rousseaux J. et al. (1986) Methods Enzymol. 121:663-669; Bird R.E. and Walker B.W. (1991) Trends Biotechnol. 9:132-137).

An antibody may be modified by conjugation with a variety of molecules, such as polyethylene glycol (PEG). The present invention provides suchmodified antibodies. A modified antibody can be obtained by chemically modifying an antibody. These modification methods are conventional in the field.

Alternatively, an antibody of the present invention may be obtained as a chimeric antibody comprising a variable region derived from a nonhuman antibody and the constant region derived from a human antibody, or as a humanized antibody comprising the complementarity determining region (CDR) derived from a nonhuman antibody, the framework region (FR) derived from a human antibody, and the constant region, by using well-known methods.

Obtained antibodies may be purified to homogeneity. Any standard method protein separation and purification method may be used for antibody separation and purification. For example, chromatographies such as affinity chromatography, filters, ultrafiltration, salting out, dialysis, SDS polyacrylamide gel electrophoresis, isoelectric point electrophoresis, and such may be appropriately combined to isolate and purify the antibody (Antibodies: A Laboratory Manual. Ed Harlow and David Lane, Cold Spring Harbor Laboratory, 1988). However, the methods are not limited thereto. The concentration of the obtained antibody may be determined by measuring absorbance, by enzyme-linked immunosorbent assay (ELISA), etc.

Columns used for affinity chromatography include, protein A column and protein G column. For example, Hyper D, POROS, Sepharose F.F. (Pharmacia), and such may be mentioned as columns using protein A columns.

Chromatographies other than affinity chromatography are, for example, ion exchange chromatography, hydrophobic chromatography, gel filtration chromatography, reverse phase chromatography, adsorption chromatography, and so on (Strategies for Protein Purification and Characterization: A Laboratory Course Manual. Ed Daniel R. Marshak et al., Cold Spring Harbor Laboratory Press, 1996). These chromatographies can be conducted using liquid chromatographies such as HPLC, and FPLC.

For example, measurement of absorbance, enzyme-linked immunosorbent assay (ELISA), enzyme immunoassay (EIA), radioimmunoassay (RIA) , or immunofluorescence may be used to measure the antigen binding activity of an antibody of the invention. In ELISA, an antibody of the present invention is immobilized on a plate, a protein of the invention is applied, and then a sample containing a desired antibody, such as culture supernatant of antibody producing cells or a purified antibody, is applied. Then, a secondary antibody labeled with an enzyme such as alkaline phosphatase that recognizes the primary antibody is applied, and the plate is incubated. After washing, an enzyme substrate, such as p-nitrophenyl phosphate, is added to the plate, and the absorbance is measured to evaluate the antigen binding activity of the sample. A fragment of a protein, such as the C-terminus fragment, may be used as the protein. BIAcore (Pharmacia) may be used to evaluate the activity of an antibody according to the present invention.

The above methods allow the detection or measurement of a protein of the invention, by exposing an antibody of the invention to a sample assumed to contain the protein of the invention, and detecting or measuring the immune complex formed by the antibody and the protein. Because the method of detection or measurement of a protein according to the invention can specifically detect or measure a protein, the method may be useful in a variety of experiments in which the protein is used.

The present invention also provides DNA comprising at least 15 nucleotides that is complementary to a DNA encoding a "DEC2" protein (SEQ ID NO: 1, 11, or 13) or to the complementary strand thereof.

"Complementary strand" herein refers to one strand of a double strand DNA comprising A:T and G:C base pairs, when viewed against the other strand. Furthermore, "complementary" means not only when a nucleotide sequence is completely complementary to a continuous nucleotide sequence with at least 15 nucleotides, but also when there is a homology of at least 70%, preferably at least 80%, more preferably 90%, and much more preferably 95% or more at the nucleotide sequence level. Homology can be determined by using the algorithm described herein.

Such DNA include probes and primers used for the detection and amplification of a DNA encoding a protein of the present invention, nucleotides and nucleotide derivatives (for example, antisense oligonucleotides or DNA encoding ribozymes, etc.) used for repressing the expression of a protein of the present invention. Furthermore, such DNA can be used in the preparation of DNA chips.

If the DNA is used as a primer, the 3' region thereof may be the complementary site, and restriction enzyme recognition sites, tag sequences, and such may be attached to the 5' region.

Antisense oligonucleotides comprise, for example, an antisense oligonucleotide that hybridizes with any portion of the nucleotide sequence of SEQ ID NO: 1, 11, or 13. The antisense oligonucleotide is preferably an antisense of a continuous sequence comprising at least 15 nucleotides or more within the nucleotide sequence of SEQ ID NO: 1, 11, or 13. More preferably, the above continuous sequence comprising at least 15 nucleotides or more contains a translation initiation codon.

A derivative or modified form of an antisense oligonucleotide may also be used. The latter form may be prepared by modifying an antisense oligonucleotide with lower alkylphosphonates such as methylphosphonate or ethylphosphonate, or with phosphorothioate, or phosphoroamidate.

The antisense oligonucleotide is not restricted to one in which all nucleotides are complementary to the corresponding nucleotides within a given region of a DNA or mRNA. As long as it can specifically hybridize with the nucleotide sequences of SEQ ID NO: 1, 11, or 13, it may have one or more nucleotide mismatches.

A derivative of an antisense oligonucleotide of the present invention may act on cells producing a protein of the invention and bind to a DNA or mRNA encoding the protein, and then, it may inhibit the expression of the protein of the invention by inhibiting its transcription or translation, or by promoting the degradation of mRNA, and thereby inhibiting the function of the protein.

A derivative of an antisense oligonucleotide of the present invention may be mixed with an appropriate base that is inactive against the derivative, and used as a medicine for external application, such as a salve or poultice.

If necessary, it may be mixed with an excipient, isotonizing agent, solubilizing agent, stabilizer, preservative, pain-killer, or the like, and prepared as a tablet, powder, granule, capsule, liposome capsule, injectable solution, liquid formulation, nose drops, freeze-dried agent, etc. The above may be achieved according to standard methods.

For treating patients, a derivative of an antisense oligonucleotide of the present invention may be, for example, directly applied to the affected area of a patient, or administered into blood vessels so as to finally reach the affected area. Moreover, the derivative may be encapsulated in antisense-encapsulating materials such as liposomes, poly-L-lysine, lipid, cholesterol, lipofectin, or their derivatives in order to increase durability and/or membrane permeability.

Dose of the derivative of the antisense oligonucleotide of the present invention may be appropriately adjusted depending on the patient's conditions, and a favorable amount such as 0.1 to 100 mg/kg, or more, preferably 0.1 to 50 mg/kg, may be administered.

As an antisense oligonucleotide of the present invention inhibits the expression of a protein of the invention, it is useful as an inhibitor of a biological activity of the protein of the invention. An inhibitor of expression comprising an antisense oligonucleotide of the present invention is useful due to its ability to inhibit a biological activity of a protein of the invention.

A protein of the invention may be useful for screening a compound that binds to the protein. Specifically, the protein may be used in a method of screening for the compound, such a method comprising the steps of exposing the protein of the present invention to a test sample expected to contain a compound binding to the protein, and selecting a compound having the activity of binding to the protein.

Proteins of the invention used for screening may be recombinant or natural proteins, or partial peptides. Alternatively, they may be in the form expressed on the surface of a cell, or in the form of a membrane fraction. Samples tested include, but are not limited to, cell extracts, cell culture supernatants, products of fermentation microorganisms, marine organism extracts, plant extracts, purified or crude preparations of proteins, peptides, non-peptide compounds, synthetic low-molecular weight compounds, and natural compounds. A protein of the present invention contacted with a test sample may be brought into contact with the test sample, for example, as a purified protein, as a soluble protein, in the form attached to a carrier, a fusion protein with other proteins, in the form expressed on the cell membrane, or as a membrane fraction.

Various methods known to one skilled in the art may be used as the screening method of, for example, a protein that binds to a protein of the present invention (e.g. other bHLH type transcription factors and other proteins related to transcriptional regulation) using a protein of the present invention. Such a screening can be carried out, for example, by the immunoprecipitation method. Specifically, the method can be carried out as follows. A gene encoding a protein of this invention is expressed by inserting the gene into vectors for foreign gene expression such as pSV2neo, pcDNA I, and pCD8, and expressing the gene in animal cells, etc. Any generally used promoter may be employed for the expression, including the SV40 early promoter (Rigby In Williamson (ed.), Genetic Engineering, Vol. 3. Academic Press, London, p.83-141 (1982)), EF-1 α promoter (Kim, et al. Gene 91, p. 217-223 (1990)), CAG promoter (Niwa, et al. Gene 108, p. 193-200 (1991)) , RSV LTR promoter (Cullen, Methods in Enzymology 152 , p. 684-704 (1987) ) , SR α promoter (Takebe et al., Mol. Cell. Biol. 8, p.466 (1988)), CMV immediate early promoter (Seed and Aruffo Proc. Natl. Acad. Sci. USA 84, p. 3365-3369 (1987)), SV40 late promoter (Gheysen and Fiers J. Mol. Appl. Genet. 1, p.385-394 (1982)), Adenovirus late promoter (Kaufman et al. , Mol. Cell. Biol. 9, p.946 (1989)), HSV TK promoter, etc. Transfer of a foreign gene into animal cells for expression therein can be performed by any of the following methods, including the electroporation method (Chu, G. etal., Nucl. Acid Res. 15, 1311-1326 (1987)), the calcium phosphate method (Chen, C. and Okayama, H. Mol. Cell. Biol. 7, 2745-2752 (1987)), the DEAE dextran method (Lopata, M. A. et al. Nucl. Acids Res. 12, 5707-5717 (1984) ; Sussman, D. J. and Milman, G. Mol. Cell. Biol. 4, 1642-1643 (1985)), the lipofectin method (Derijard, B. Cell. 7, 1025-1037 (1994); Lamb, B. T. et al. Nature Genetics 5, 22-30 (1993)), Rabindran, S. K. et al. Science 259, 230-234 (1993)), etc. A protein of this invention can be expressed as a fusion protein having a recognition site for a monoclonal antibody whose specificity has been established by introducing the recognition site (epitope) into the N- or C-terminal of a protein of this invention. For this purpose, a commercial epitope-antibody system can be utilized (Jikken Igaku (Experimental Medicine) 13, 85-90 (1995)). Vectors that are capable of expressing fusion proteins with β-galactosidase, maltose-binding protein, glutathione S-transferase, green fluorescence protein (GFP), and such, via a multi-cloning site are commercially available.

To minimize the alteration in properties of a protein of this invention due to fusion protein formation, a method for preparing a fusion protein by introducing only a small epitope portion comprising several to ten amino acid residues has been reported. For example, the epitopes of polyhistidine (His-tag), influenza hemagglutinin (HA), human c-myc, FLAG, Vesicular stomatitis virus glycoprotein (VSV-GP), T7 gene 10 protein (T7-tag), human herpes simplex virus glycoprotein (HSV-tag), E-tag (epitope on the monoclonal phage), and such, and monoclonal antibodies to recognize these epitopes can be utilized as the epitope-antibody system for screening proteins binding to the protein of this invention (Jikken Igaku (Experimental Medicine) 13, 85-90 (1995)).

In immunoprecipitation, immune complexes are formed by adding these antibodies to the cell lysate prepared using suitable surfactants. This immune complex comprises a protein of this invention, a protein capable of binding to the protein, and an antibody. The immunoprecipitation can also be performed using an antibody to a protein of this invention, besides antibodies to the above-described epitopes. An antibody to a protein of this invention can be prepared by, for example, inserting a gene encoding a protein of this invention into an appropriate expression vector of *E. coli* to express it in the bacterium, purifying the protein thus expressed, and immunizing rabbits, mice, rats, goats, chicken, and such, with the purified protein. The antibody can also be prepared by immunizing the above-described animals with partial peptides of a protein of this invention.

Immune complexes can be precipitated using, for example, Protein A Sepharose and Protein G Sepharose when the antibody is a murine IgG antibody. In addition, when the protein of this invention is prepared as a fusion protein with the epitope of, for example, GST, and such, the immune complex can be formed using a substance that specifically binds to these epitopes, such as glutathione-Sepharose 4B, and such, giving the same result as in the case where the antibody for the protein of this invention is used.

Immune precipitation, in general, may be carried out according to, or following the method described in literature (Harlow, E. and Lane, D.: Antibodies, pp.511-552, Cold Spring Harbor Laboratory publications, New York (1988)).

SDS-PAGE is generally used for the analysis of immunoprecipitated proteins. Bound proteins can be analyzed based on the molecular weights of proteins using a gel of an appropriate concentration. In this case, although proteins bound to the protein of this invention, are in general hardly detectable by the usual protein staining method, such as Coomassie staining and silver staining, the detection sensitivity can be improved by culturing cells in a medium containing radio isotope-labeled ³⁵S-methionine and ³⁵S-cysteine to label proteins inside the cells, and detecting the labeled proteins. Once the molecular weight of the protein is determined, the desired protein can be purified directly from SDS-polyacrylamide gel and sequenced.

Isolation of a protein that binds to a protein of the present invention using the protein may be carried out by, for example, using the West-Western blotting method (Skolnik E.Y. et al. (1991) Cell 65:83-90). Specifically, a cDNA library is constructed from cells, tissues, or organs (for example, tissues or cultured cells from brain, skeletal muscle, testis, placenta, large intestine, spleen, cartilages, and so on) in which a protein binding to a protein of the present invention is expected to be expressed, by using phage vectors (λgt11, ZAP, etc.). Then, this is expressed on LB-agarose, transferred to a filter membrane, which is reacted with a purified labeled protein of the invention. The plaques expressing proteins that bind to the protein of the invention can be identified by detecting the label. The protein of the invention may be labeled by a method utilizing the binding between biotin and avidin, or a method utilizing an antibody that specifically binds to the protein of the present invention, or a peptide or polypeptide (e.g. GST and such) that is fused to the protein of the present invention. Methods using radioisotope or fluorescence and such may also be used.

Alternatively, in another embodiment of the method for screening of the present invention, a two-hybrid system utilizing cells may be used (Fields S. and Sternglanz R. (1994) Trends Genet. 10:286-292; Dalton S. and Treisman R. (1992) "Characterization of SAP-1, a protein recruited by serum response factor to the c-fos serum response element." Cell 68:597-612; "MATCHMAKER Two-Hybrid System", "Mammalian MATCHMAKER Two-Hybrid Assay Kit", "MATCHMAKER One-Hybrid System" (products of Clontech); "HybriZAP Two-Hybrid Vector System" (Stratagene)). The two-hybrid system can be used as follows: (1) a protein of the present invention or a partial peptide thereof is fused to the SRF DNA binding region or GAL4 DNA binding region and expressed in yeast cells; (2) a cDNA library, which expresses proteins as fusion proteins with VP16 or GAL4 transcription activating regions, is prepared from cells expected to express proteins binding to the protein of the present invention; (3) the library is introduced to above mentioned yeast cells; and (4) library-derived cDNA are isolated from the positive clones detected (positive clones can be confirmed by activation of reporter genes due to the binding of the present protein and the binding protein expressed in the yeast cell). The protein encoded by the cDNA can be obtained by introducing the isolated cDNA into *E.coli* and expressing it. Thus, a protein binding to a present protein or genes thereof can be prepared. For example, in addition the HIS3 gene, Ade2 gene, LacZ gene, CAT gene, luciferase gene, PAI-1 (Plasminogen activator inhibitor typel) gene, and so on, can be mentioned as reporter genes used in the 2-hybrid system, but are not restricted thereto.

Alternatively, a compound binding to a protein of the present invention can be screened by affinity chromatography. For example, a protein of the invention is immobilized on a carrier of an affinity column, and a test sample, in which a protein capable of binding to the protein of the invention is presumed to be expressed, is applied to the column. The test sample used herein may be a cell extract, cell lysate, etc. After loading the test sample, the column is washed, and a protein bound to the protein of the invention can be obtained.

The DNA encoding the protein may be obtained by analyzing the amino acid sequence of the obtained protein, synthesizing oligo DNA based on the sequence information, and screening a cDNA library using the DNA as the probe.

A biosensor utilizing the surface plasmon resonance phenomenon may be used as a means for detecting or measuring a compound bound to the present invention. When such a biosensor is used, the interaction between a protein of the invention and a test compound can be observed at real-time as a surface plasmon resonance signal, using only a minute amount of proteins without labeling (for example, BIAcore, Pharmacia) . Therefore, it is possible to evaluate the binding between a protein of the invention and a test compound using a biosensor such as BIAcore.

In addition, methods for isolating not only proteins, but also compounds binding to the proteins of the invention (including agonists and antagonists) are known in the art. Such methods include, for example, the method of screening for a binding molecule by contacting synthesized compounds or natural substance banks, or random phage peptide display libraries with an immobilized protein of the invention, and the high-throughput screening method using the combinatorial chemistry technique (Wrighton, N. C., Farrell, F. X., Chang R., Kashyap A. K., Barbone F. P., Mulcahy L. S., Johnson D. L., Barrett R. W., Jolliffe L. K., and Dower W. J., "Small peptides as potent mimetics of the protein hormone erythropoietin" Science (UNITED STATES), Jul 26, 1996, 273 p458-64; Verdine G.L., "The combinatorial chemistry of nature" Nature (ENGLAND), Nov 7, 1996, 384, p11-13; Hogan J. C. Jr., "Directed combinatorial chemistry" Nature (ENGLAND) Nov 7, 1996, 384, p17-9)

A compound that binds to a protein of the present invention may be a candidate for a drug that can promote or inhibit the activity of the protein, and thus may be applied to the treatment of diseases caused by an abnormal expression, function, and such of the protein. Such a drug may also be applied against diseases that may be treated by controlling the activity of a protein of the present invention. Compounds, which are obtained by the screening method of this invention and which have the activity to bind to a protein of this invention whose partial structure is modified by an addition, deletion and/or substitution, are also included in compounds that bind to the proteins of this invention.

When using a compound binding to a protein of this invention, or a protein of this invention as a pharmaceutical agent for humans and other mammals, such as mice, rats, guinea-pigs, rabbits, chicken, cats, dogs, sheep, pigs, cattle, monkeys, baboons, and chimpanzees, the protein or the isolated compound can be directly administered or can be formulated using known pharmaceutical preparation methods. For example, according to the need, the drugs can be taken orally as sugarcoated tablets, capsules, elixirs and microcapsules or non-orally in the formof injections of sterile solutions or suspensions with water or any other pharmaceutically acceptable liquid. For example, the compounds can be mixed with pharmacologically acceptable carriers or medium, specifically, sterilized water, physiological saline, plant-oil, emulsifiers, solvents, surfactants, stabilizers, flavoring agents, excipients, vehicles, preservatives and binders, in a unit dose form required for generally accepted drug implementation. The amount of active ingredients in these preparations makes a suitable dosage within the indicated range acquirable.

Examples of additives that can be mixed to tablets and capsules are, binders such as gelatin, corn starch, tragacanth gum and arabic gum; excipients such as crystalline cellulose; swelling agents such as corn starch, gelatin and alginic acid; lubricants such as magnesium stearate; sweeteners such as sucrose, lactose or saccharin; flavoring agents such as peppermint, Gaultheria adenothrix oil and cherry. When the unit dosage form is a capsule, a liquid carrier, such as oil, can also be included in the above ingredients. Sterile composites for injections can be formulated following normal drug implementations using vehicles such as distilled water used for injections.

Physiological saline, glucose, and other isotonic liquids including adjuvants, such as D-sorbitol, D-mannnose, D-mannitol, and sodium chloride, can be used as aqueous solutions for injections. These can be used in conjunction with suitable solubilizers, such as alcohol, specifically ethanol, polyalcohols such as propylene glycol and polyethylene glycol, non-ionic surfactants, such as Polysorbate 80 (TM) and HCO-50.

Sesame oil or Soy-bean oil can be used as a oleaginous liquid and may be used in conjunction with benzyl benzoate or benzyl alcohol as a solubilizer; may be formulated with a buffer such as phosphate buffer and sodium acetate buffer; a pain-killer such as procaine hydrochloride; a stabilizer such as benzyl alcohol, phenol; and an anti-oxidant. The prepared injection is filled into a suitable ampule.

Methods well known to one skilled in the art may be used to administer a pharmaceutical compound to patients, for example as intraarterial, intravenous, subcutaneous injections and also as intranasal, transbronchial, intramuscular, percutaneous, or oral administrations. The dosage varies according to the body-weight and age of a patient, and the administration method, but one skilled in the art can suitably select the dosage. If said compound can be encoded by a DNA, the DNA can be inserted into a vector for gene therapy to perform the therapy. The dosage and method of administration vary according to the body-weight, age, and symptoms of a patient, but one skilled in the art can select them suitably.

The dose of the protein of the invention may vary depending on the subject, target organ, symptoms, and administration method, but may be, in general, about 100 µg to 20 mg per day for a normal adult (body weight: 60 kg).

Although varying according to the symptoms, the dose of a compound that binds to a protein of this invention or a compound that inhibits the activity of the protein are generally in the range of about 0.1 to 100 mg, preferably about 1.0 to 50 mg, and more preferably about 1.0 to 20 mg per day for adults (body weight: 60 kg) in the case of an oral administration.

Although varying according to the subject, target organ, symptoms, and method of administration, a single dose of a compound for parenteral administration is preferably, for example. when administered-intravenously to normal adults (60kg body weight) in the form of injection, in the range of about 0.01 to 30 mg, preferably about 0.1 to 20 mg, and more preferably about 0.1 to 10 mg per day. Doses converted to 60 kg body weight or per body surface area can be administered to other animals.

### Brief Description of the Drawings

Figure 1 shows an alignment of the amino acid sequences of human DEC2a (hDEC2a) and DEC1.
Figure 2 shows an alignment of the amino acid sequences of human DEC2a (hDEC2a) and human DEC2b (hDEC2b).
Figure 3 shows an alignment of the amino acid sequences of human DEC2a (hDEC2a) and mouse DEC2a (mDEC2a).
Figure 4 shows an alignment of the amino acid sequences of human DEC2a (hDEC2a), mouse DEC2a (mDEC2a), and SHARP-1.

### Best Mode for Carrying out the Invention

The present invention is described in detail hereinafter using examples, but it is not to be construed as being limited to them.

### [Example 1] Isolation of novel DEC2 genes belonging to the DEC1 subfamily

Using the full-length nucleotide sequence of DEC1 cDNA coding region, the human EST database was searched for cDNA fragments having similarity to DEC1. The full-length cDNA nucleotide sequence of one cDNA fragment selected from the several cDNA fragments searched was determined according the 3'-RACE and 5'-RACE method.

1 µg cDNA synthesized from human skeletal muscle mRNA using SuperScript Preamplification System (Gibco BRL) was used as the template in the first phase PCR. PCR was performed using LA taq (TaKaRa), with a full volume of 50 µl containing 400 µM of each dNTP and 0.2 µM of each primer under the following conditions: 94°C for 1 min, followed by 30 cycles of 98°C for 20 sec and 68°C for 3 min, and finally, 72°C for 5 min. Thereafter, 5 µl of 1/25 diluents of the reaction solution was used as the template, and second phase PCR was conducted under the same condition as in the first phase. Primers used were as follows : ACT2-5' (5'-CTATTCGATGATGAAGATACCCCACCAAACCC-3'/SEQ ID NO:3) and 3'006A (5'-GCAAGTGGTTGATCAGCTGGACACA-3'/SEQ ID NO:4) for the first phase of 5'-RACE; ACT2-B (5'-GCTTACCCATACGATGTTCCA-3'/SEQ ID NO:5) and 5'006A (5'-TGGAACGCATCCAAGTCGGACTGAAT-3'/SEQ ID NO:6) for the second phase of 5'-RACE; 065'S2 (5'-TTGAACATGGACGAAGGAATTCC-3'/SEQ ID NO:7) and ACT2-3' (5'-GAGATGGTGCACGATGCACAGTTGAAGTGAAC-3'/SEQ ID NO:8) for the first phase of 3'-RACE; and 5'006S (5'-ATTCAGTCCGACTTGGATGCGTTCCA-3'/SEQ ID NO:9) and ACT2-3'2 (5'-GCGGGGTTTTTCAGTATCTACGA-3'/SEQ ID NO:10) for the second phase of 3'-RACE.

The PCR products of the second phase were separated by 1% agarose gel electrophoresis, bands were cut out from the gel, and DNA fragments were extracted using GENECLEAN II kit (BIO101). Obtained DNA fragments were cloned into pGEM-T Easy vector (Promega) by TA cloning. After isolating the clones, plasmids were purified and used for sequencing. Sequences were analyzed using ABI PRISM 310 DNA Analyzer (PE Applied Biosystems) after reacting with BigDye Terminator Cycle Sequencing Ready Reaction (PE Applied Biosystems). Clones obtained from at least three independent PCRs were sequenced and the nucleotide sequences were determined.

Two DEC1 subfamily genes thus identified were named DEC2a and DEC2b. The determined nucleotide sequence of DEC2a cDNA and the amino acid sequence of the protein encoded by the cDNA are shown in SEQ ID NOs : 1 and 2, respectively. Furthermore, the determined nucleotide sequence of human DEC2b and amino acid sequence of the protein encoded by the cDNA are shown in SEQ ID NOs: 11 and 12, respectively.

Then, the present inventors isolated mouse cDNA corresponding to the isolated human cDNA. First, the mouse EST database was searched for cDNA fragments having similarity to human DEC2a and human DEC2b cDNA using the full-length nucleotide sequences of human DEC2a and 2b cDNA. Then, cDNA fragments, which were presumed to contain the upstream region of the protein translation initiation site or the downstream region of the protein translation termination site, were selected from the searched cDNA fragments, and primers were designed to recover the full-length coding region by PCR. Designed primer sequences were as follows: 5'-AAAATCTCTCCAGGCGACCGT-3' (SEQ ID NO: 15) and 5'-AGCCTGTCGAGCATCGCTTA-3' (SEQIDNO: 16) .Using this primer pair and the cDNA prepared from mouse skeletal muscle mRNA as the template, PCR was conducted under the same conditions as described above. As a result of nucleotide sequence determination of the amplified cDNA, it was revealed that a mouse cDNA with a similar sequence to the human DEC2a had been obtained. This clone was named "mouse DEC2a". The nucleotide sequence of mouse DEC2a and amino acid sequence of the protein encoded by the cDNA are shown in SEQ ID NOs: 13 and 14, respectively.

### [Example 2] Structural analysis of DEC2

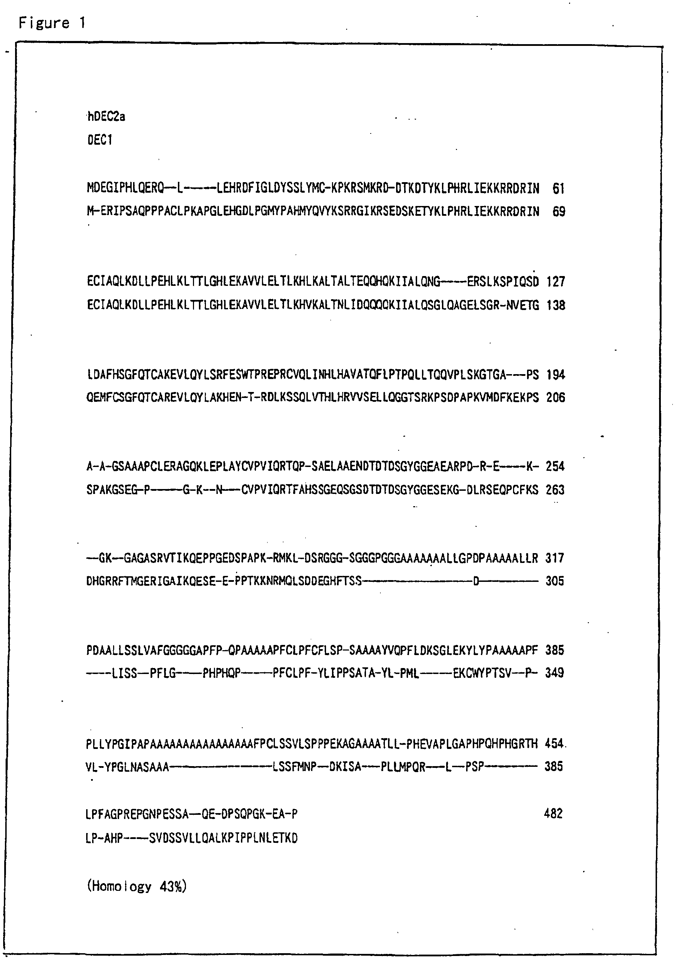

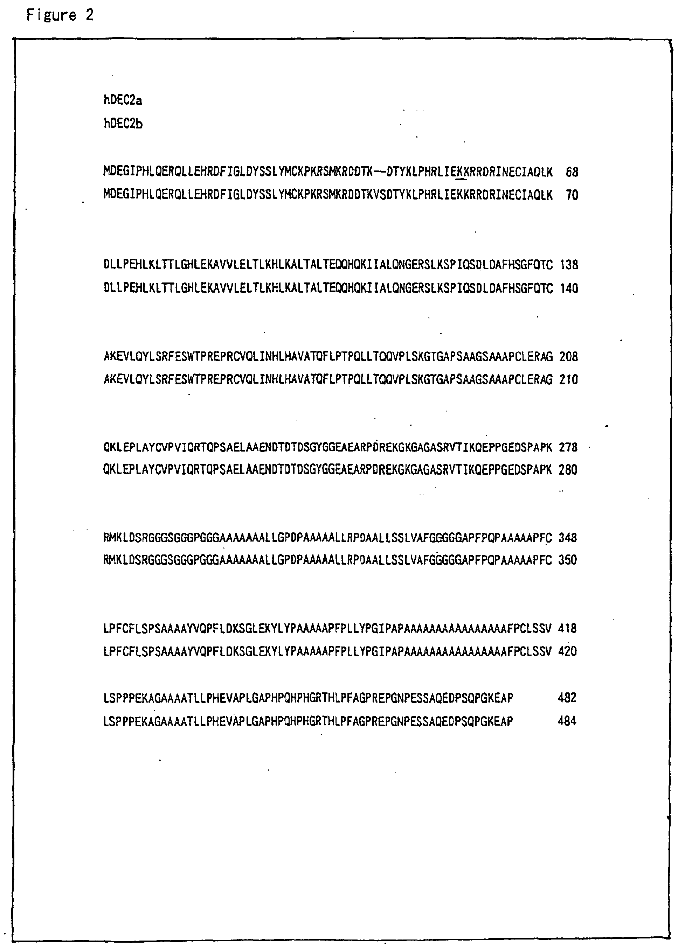

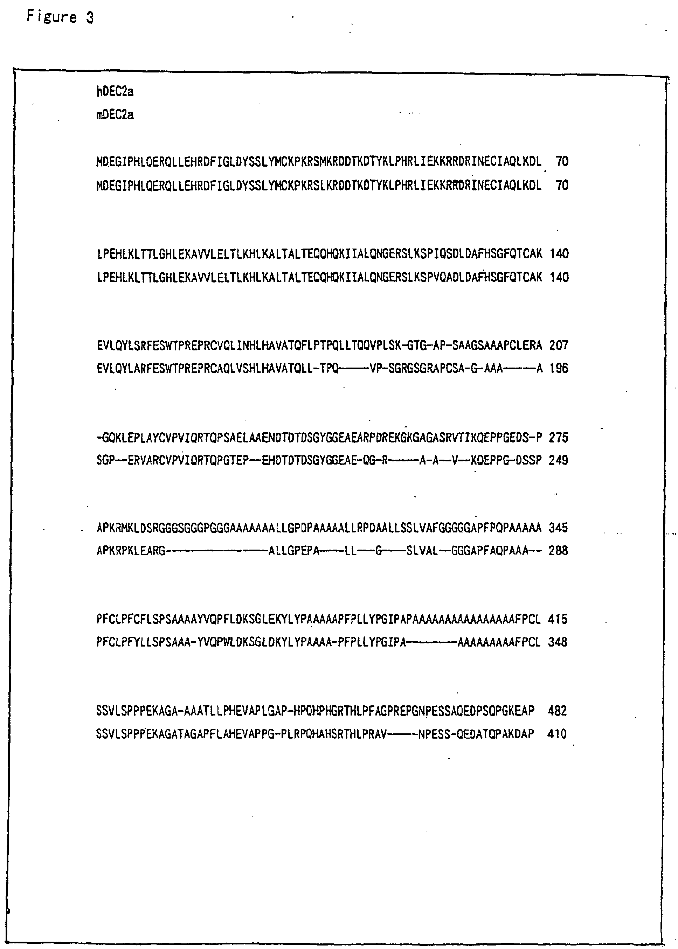

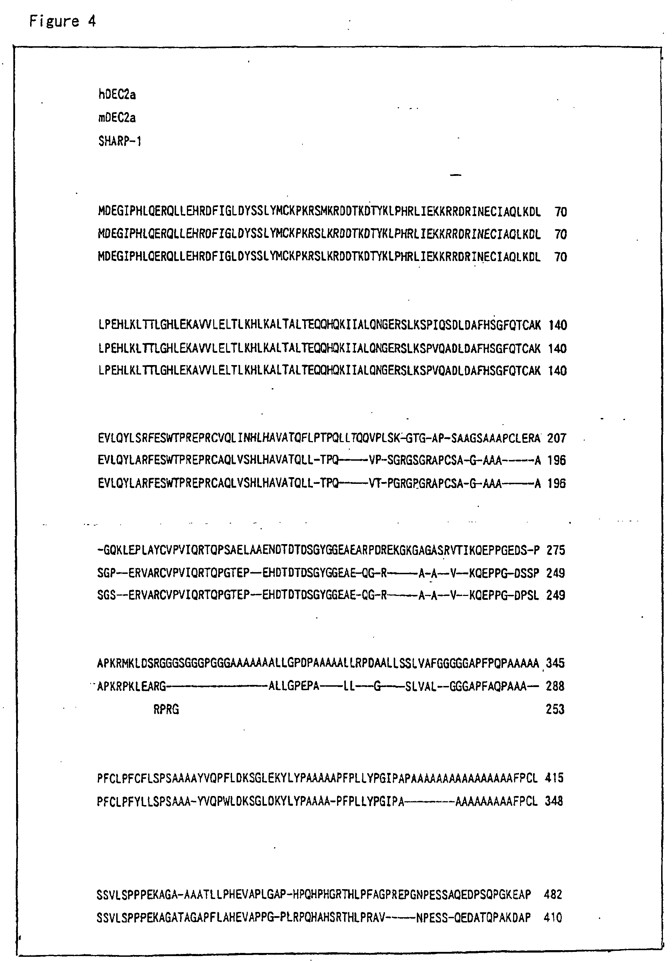

The nucleotide sequence of human DEC2a cDNA had an open reading frame encoding a protein consisting of 482 amino acids. The protein had a basic Helix-Loop-Helix (bHLH) structure, and was supposed to be a bHLH type transcription factor. According to the alignment analysis with the DEC1 protein, an especially high amino acid sequence similarity was detected in the bHLH region (Figure 1). The structures of human DEC2b and human DEC2a have a high resemblance to each other. and DEC2b had a structure in which 2 amino acids (valine and serine) were inserted immediately before the basic region of the DEC2a bHLH structure. These two are thought to be isoforms of one another (Figure 2). The nucleotide sequence of mouse DEC2a cDNA had an open reading frame encoding a protein consisting of 410 amino acids, and had a homology of 73% at the amino acid level to human DEC2a (Figure 3). Mouse DEC2a, human DEC2a and human DEC2b proteins showed the highest homology to the amino acid sequence of rat SHARP protein, and the homology was especially high in the N-terminus half that includes the bHLH region (Figure 4). It was revealed, however, that the homology between regions of the C-terminus half was relatively low.

### Industrial Applicability

The present invention provides novel bHLH type transcription factors and their genes. Since the genes are thought to be involved in the differentiation and proliferation of cells and tissues, they may be used as factors controlling these cellular functions. Furthermore, they may be used in the purification and cloning of novel factors related to development and cell proliferation, and as tools for developing pharmaceutical agents for various diseases caused by abnormal expression of the genes of the present invention due to abnormal regulation of expression *in vivo.* Designing drugs and such that target genes of the present invention may enable the development of pharmaceutical agents with novel functional mechanisms.

## Claims

1. A DNA according to any one of the following (a) to (d):
(a) a DNA comprising the coding region of the nucleotide sequence of SEQ ID NO: 1, 11, or 13,
(b) a DNA encoding a protein comprising the amino acid sequence of SEQ ID NO: 2, 12, or 14,
(c) a DNA encoding a protein comprising an amino acid sequence in which one or more amino acids in the amino acid sequence of SEQ ID NO: 2, 12, or 14 has been substituted, deleted, inserted, and/or added, wherein the protein is functionally equivalent to the protein comprising the amino acid sequence of SEQ ID NO: 2, 12, or 14, and,
(d) a DNA that hybridizes under stringent conditions to a DNA comprising the nucleotide sequence of SEQ ID NO: 1, 11, or 13, and encodes a protein that is functionally equivalent to the protein comprising the amino acid sequence of SEQ ID NO:2, 12, or 14.

2. A DNA that encodes a partial peptide of the protein of SEQ ID NO: 2, 12, or 14.

3. A vector into which a DNA of claim 1 or 2 is inserted.

4. A transformant carrying a DNA of claim 1 or 2, or the vector of claim 3.

5. A protein or peptide encoded by a DNA of claim 1 or 2.

6. Amethod for producing the protein or peptide of claim 5, comprising the steps of:
culturing the transformant of claim 4, and,
recovering the expressed protein from the transformant or culture supernatant.

7. A DNA comprising at least 15 nucleotides, wherein the DNA is complementary to a DNA comprising the nucleotide sequence of SEQ ID NO: 1, 11, or 13, or to the complementary strand thereof.

8. A method of screening for a compound that binds to the protein of claim 5, comprising the steps of:
(a) contacting a test sample with said protein or partial peptide,
(b) detecting the binding activity of said protein or partial peptide with the test sample, and,
(c) selecting a compound that has a binding activity to said protein or partial peptide.

9. A compound that binds to the protein of claim 5.

10. The compound of claim 9, wherein said compound is an antibody.

11. The compound of claim 9, wherein said compound is isolated by a method as set forth in claim 8.
